# EUROPEAN PATENT SPECIFICATION

(11) **EP 2 948 074 B1**
(45) Date of publication and mention of the grant of the patent: **19.09.2018**
(21) Application number: 14706985.0
(22) Date of filing: 24.01.2014
(51) Int. Cl.: A61B 17/24, A61B 34/20, H05K 1/03

(54) **SURGICAL INSTRUMENT WITH TRACKING DEVICE CONNECTED VIA A FLEXIBLE CIRCUIT**
CHIRURGISCHES INSTRUMENT MIT EINER ÜBER EINE FLEXIBLE SCHALTUNG VERBUNDENEN VERFOLGUNGSVORRICHTUNG
INSTRUMENT CHIRURGICAL À DISPOSITIF DE SUIVI CONNECTÉ PAR LE BIAIS D'UN CIRCUIT SOUPLE

(30) Priority: 25.01.2013 US 201313751032
(43) Date of publication of application: 02.12.2015
(73) Proprietor: Medtronic Xomed, Inc., Jacksonville, FL 32216 (US)
(72) Inventor: JACOBSEN, Brad, Erie, Colorado 80516 (US); BURG, Bruce M., Louisville, Colorado 80027 (US); BLOCK, Orey G., Westminster, Colorado 80031 (US); BZOSTEK, Andrew, Boulder, Colorado 80301 (US); DOERR, Vince J., Boulder, Colorado 80305 (US); JAIN, Abhishek, Centennial, Colorado 80016 (US); MERKL, Brandon, Erie, Colorado 80516 (US); CILKE, Joseph Thomas, Broomfield, Colorado 80023 (US)
(74) Representative: Dehns
(86) International application number: PCT/US2014/012967
(87) International publication number: WO 2014/116961

(56) References cited:
- WO-A1-2008/105874
- US-A1- 2011 270 081
- US-A1- 2013 137 954
- "Minco Bulletin FC-3", , 31 July 2002 (2002-07-31), XP055115671, Retrieved from the Internet: URL:http://www.temflexcontrols.com/pdf/fc3 .pdf [retrieved on 2014-04-29]

## Description

### FIELD

The present disclosure relates generally to a flexible circuit sheet and, more particularly, a flexible circuit sheet for a surgical instrument.

### BACKGROUND

The statements in this section merely provide background information related to the present disclosure and may not constitute prior art.

Surgical procedures can be performed on anatomies such as the human anatomy for providing a therapy to the anatomy. One area of surgery includes procedures performed on facial cavities of a patient such as on the ear, nose or throat (ENT). In such a procedure, a surgical instrument such as a suction device may be inserted into such a cavity to perform a procedure for example. Because the viewing angle of a surgeon at the area of interest can be obscured by the surrounding tissue of the cavity, the ability of a surgeon to effectively apply a therapy, such as a suction procedure, can be reduced. In some procedures, it may also be difficult to effectively guide the surgical instrument through various shaped cavities of the anatomy. In an effort to address this difficulty, instruments have been developed that include flexible elongated portions configured to be permanently flexible. While these flexible instruments can conform to internal cavities of the anatomy, they do not retain any specific configuration, such that they are generally not suitable for certain procedures, such as an ENT suction procedure.

In navigation systems, instruments are provided with tracking devices. Sometimes, however, such tracking devices can be difficult to manipulate or cumbersome to couple to the instrument, especially instruments with the flexible elongated portions. For example, it can be difficult to electrically couple the tracking devices to associated lead wires relative to the flexible elongated portion. In other instances, the tracking devices can be positioned in a handle or proximal region of the instrument such that if the distal tip moves or is moved relative to the handle, the distal tip can no longer be accurately tracked.

### SUMMARY

This section provides a general summary of the disclosure, and is not a comprehensive disclosure of its full scope or all of its features.

A surgical instrument, according to the present teaching has an elongated body portion having a proximal end and a distal end. The body portion has an inner diameter defining a first internal flow passage between the proximal and distal ends, and is formed from a malleable metallic material such that the body portion can be bent between the proximal and distal ends from a first configuration to a second bent configuration and maintain the bent configuration. A handle portion coupled to the proximal end of the body portion and including a second internal passage in fluid communication with the first internal flow passage. A tracking device positioned adjacent the distal end and adapted to cooperate with a navigation system to track the location of a distal tip of the instrument, and including. A flexible circuit is disposed around the body portion from the tracking device to the handle portion, the flexible circuit configured to conform to the bent configuration of the body portion such that they do not strain or break during bending of the body portion.

Further according to the present teachings, a surgical instrument is provided having of an elongated body portion having a proximal end and a distal end. A tracking device is coupled to the elongated tubular body portion adjacent to the distal end. The tracking device is adapted to cooperate with a navigation system and includes a flexible circuit disposed about the tubular body portion between the proximal and distal ends.

Further areas of applicability will become apparent from the description provided herein. It should be understood that the description and specific examples are intended for purposes of illustration only and are not intended to limit the scope of the present teachings.

### DRAWINGS

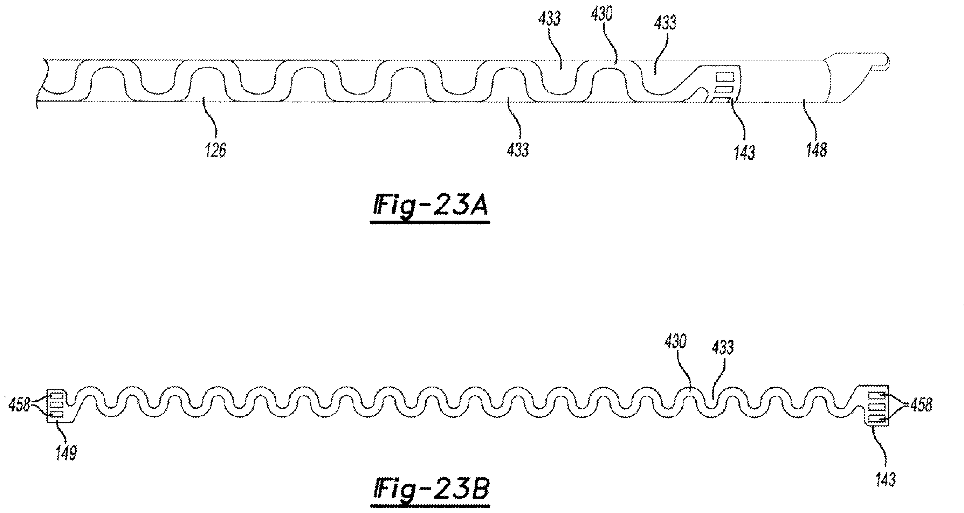

The present teachings will become more fully understood from the detailed description, the appended claims and the following drawings. The drawings are for illustrative purposes only of selected embodiments and alternative variants, and are not intended to limit the scope of the present disclosure. The flexible circuit sheet curved around a same side of the elongate body of the instrument, as claimed in the present invention, is shown in figure 23A.
Figure 1 is a perspective view of an exemplary navigation system according to the principles of the present disclosure;
Figure 2 is a top plan view of an exemplary malleable suction instrument for use with the navigation system according to the principles of the present disclosure;
Figure 3 is a side view of the exemplary suction instrument according to the principles of the present disclosure;
Figure 4 is a partial perspective view of a distal region of the exemplary suction instrument having an exemplary flexible circuit sheet according to the principles of the present disclosure;
Figure 5 is a partial side view of the distal region of the exemplary suction instrument associated with the exemplary flexible circuit sheet according to the principles of the present disclosure;
Figure 5A is an exploded view of an exemplary wire routing configuration according to the principles of the present disclosure;
Figure 6 is a partial sectional view of the exemplary suction instrument of Figure 5 according to the principles of the present disclosure;
Figure 7 is a partial view of a handle portion of the exemplary suction instrument according to the principles of the present disclosure;
Figures 8 and 9 illustrate views of exemplary alternative tracking sensor configurations according to the principles of the present disclosure;
Figure 10 is a view of exemplary bent or formed configurations of the exemplary malleable suction instrument according to the principles of the present disclosure;
Figure 11 is a partial perspective view of the distal region of the exemplary suction instrument illustrating an exemplary alternative tracking arrangement associated with the exemplary flexible circuit sheet according to the principles of the present disclosure;
Figure 12 is a partial perspective view of the distal region of the exemplary suction instrument illustrating another exemplary alternative tracking arrangement according to the principles of the present disclosure;
Figure 13A is an exploded perspective view of an exemplary configuration of the flexible printed circuit sheet according to the principles of the present disclosure;
Figure 13B is a perspective view of the flexible printed circuit sheet of Figure 13A in an exemplary assembled configuration according to the principles of the present disclosure;
Figure 14 is a perspective view of another exemplary flexible printed circuit sheet according to the principles of the present disclosure;
Figure 15 is a perspective view illustrating the flexible printed circuit sheet of Figure 14 in a bent or flexed condition according to the principles of the present disclosure;
Figure 16 is a perspective view of the flexible printed circuit sheet of Figures 14 and 15 in a flexed condition conforming to an outer surface of an exemplary instrument according to the principles of the present disclosure;
Figure 17 is a partial side view of the distal region of the exemplary suction instrument of Figure 5 associated with the exemplary flexible circuit sheet and having wire management channels according to the principles of the present disclosure;
Figure 18 is a perspective view of a patient tracking device having an exemplary flexible printed circuit sheet and associated coils according to the principles of the present disclosure;
Figure 19 is a top view of another exemplary flexible printed sheet according to the principles of the present disclosure;
Figures 20A-20C are side views representing various exemplary configurations of the flexible printed circuit sheet of Figure 19 according to the principles of the present disclosure;
Figure 21 is a top plane view of an exemplary surgical instrument for use with a navigation system according to the principles of the present disclosure;
Figure 22 represents a top view of traces associated with the flexible circuit sheet of Figure 23B;
Figure 23A represents a malleable suction tube shown in Figure 21;
Figure 23B represents a conformed flexible circuit shown in Figure 22A;
Figures 24A-24O represent perspective views of these exemplary configurations of a flexible circuit sheet according to the present disclosure; and
Figures 25 and 26 represent cross-sectional views of the flexible circuit sheet shown in Figures 24A through 24O.

### DESCRIPTION OF THE VARIOUS EMBODIMENTS OF THE DISCLOSURE

The following description is merely exemplary in nature and is not intended to limit the present disclosure, its application, or uses. It should be understood that throughout the drawings, corresponding reference numerals indicate like or corresponding parts and features with the various elements in each view being drawn to scale. Although the following description is related generally to a flexible circuit sheet operatively associated with an exemplary flexible or malleable suction instrument, it will be appreciated that the flexible circuit sheet can be associated with various devices and/or instruments, including various other surgical instruments.

Various exemplary embodiments of the disclosure are provided so that this disclosure will be thorough, and will fully convey the scope to those who are skilled in the art. Numerous specific details are set forth such as examples of specific components, devices, systems and/or methods, to provide a thorough understanding of exemplary embodiments of the present disclosure. It will be apparent to those skilled in the art that specific details need not be employed, that exemplary embodiments may be embodied in many different forms and that neither should be construed to limit the scope of the disclosure. In some exemplary embodiments, well-known processes, well-known device structures, and well-known technologies are not described in detail.

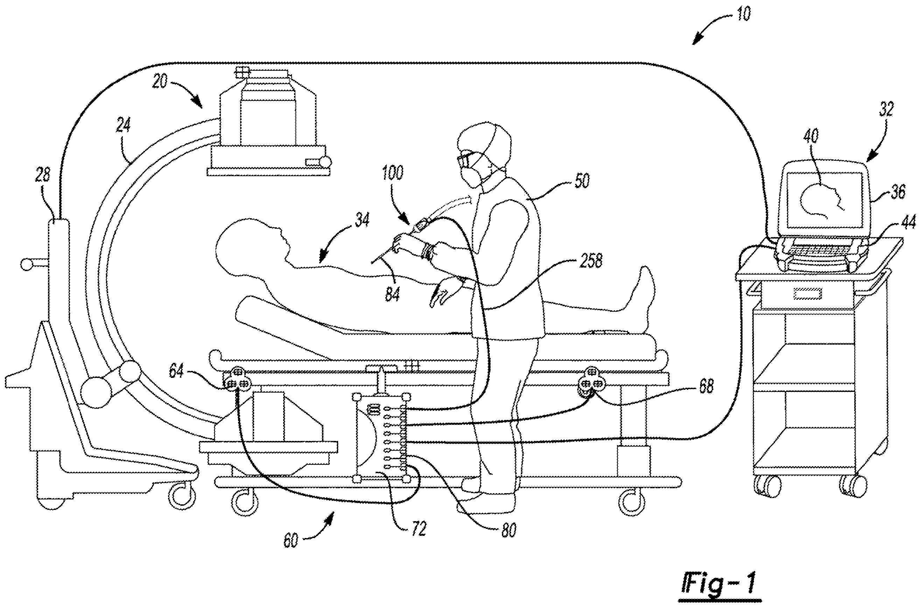

Figure 1 is a diagram schematically illustrating an overview of an image-guided navigation system 10 for use in the non-line-of-site navigating of a surgical instrument 100, such as a navigable malleable suction device or suction instrument, according to various exemplary embodiments of the present disclosure. Exemplary navigation systems include those disclosed in U.S. Pat. No. 7,366,562, issued on April 29, 2008 to John H. Dukesherer et al. and U.S. Pat. App. Pub No. 2008/0132909, published June 5, 2008, to Bradley A. Jascob et al. Commercial navigation systems include the StealthStation® AxiEM™ Surgical Navigation System sold by Medtronic Navigation, Inc. having a place of business in Louisville, Colorado, USA. It should be appreciated that while the navigation system 10 and suction instrument 100 are generally described in connection with an ear, nose and throat (ENT) procedure, navigation system 10 and suction instrument 100 can be used in various other appropriate procedures.

Generally, the navigation system 10 can be used to track a location of an exemplary suction instrument 100, including a distal tip or end thereof, that includes an exemplary flexible printed circuit sheet 232 associated therewith, as will be described herein. Navigation system 10 can generally include an optional imaging system 20, such as a fluoroscopic X-ray imaging device configured as a C-arm 24 and an image device controller 28. The C-arm imaging system 20 can be any appropriate imaging system, such as a digital or CCD camera, which are well understood in the art. Image data obtained can be stored in the C-arm controller 28 and sent to a navigation computer and/or processor controller or work station 32 having a display device 36 to display image data 40 and a user interface 44. The work station 32 can also include or be connected to an image processor, navigation processor, and a memory to hold instruction and data. The work station 32 can include an optimization processor that assists in a navigated procedure. It will also be understood that the image data is not necessarily first retained in the controller 28, but may also be directly transmitted to the workstation 32. Moreover, processing for the navigation system and optimization can all be done with a single or multiple processors all of which may or may not be included in the work station 32.

The work station 32 provides facilities for displaying the image data 40 as an image on the display device 36, saving, digitally manipulating, or printing a hard copy image of the received image data. The user interface 44, which may be a keyboard, mouse, touch pen, touch screen or other suitable device, allows a physician or user 50 to provide inputs to control the imaging device 20, via the C-arm controller 28, or adjust the display settings of the display device 36.

With continuing reference to Figure 1, the navigation system 10 can further include a tracking system, such as an electromagnetic (EM) tracking system 60. The discussion of the EM tracking system 60 can be understood to relate to any appropriate tracking system. The EM tracking system 60 can include a localizer, such as a coil array 64 and/or second coil array 68, a coil array controller 72, a navigation probe interface 80, and the trackable suction instrument 100. Instrument 100 can include an instrument tracking device or devices 84, as will be discussed herein. Briefly, the tracking device 84 can include an electromagnetic coil to sense a field produced by the localizing coil arrays 64, 68 and provide information to the navigation system 10 to determine a location of the tracking device 84. The navigation system 10 can then determine a position of a distal tip of the suction instrument 100 to allow for navigation relative to the patient 34 and patient space.

The EM tracking system 60 can use the coil arrays 64, 68 to create an electromagnetic field used for navigation. The coil arrays 64, 68 can include a plurality of coils that are each operable to generate distinct electromagnetic fields into the navigation region of the patient 34, which is sometimes referred to as patient space. Representative electromagnetic systems are set forth in U.S. Patent No. 5,913,820, entitled "Position Location System," issued June 22, 1999 and U.S. Patent No. 5,592,939, entitled "Method and System for Navigating a Catheter Probe," issued January 14, 1997.

The coil arrays 64, 68 can be controlled or driven by the coil array controller 72. The coil array controller 72 can drive each coil in the coil arrays 64, 68 in a time division multiplex or a frequency division multiplex manner. In this regard, each coil may be driven separately at a distinct time or all of the coils may be driven simultaneously with each being driven by a different frequency.

Upon driving the coils in the coil arrays 64, 68 with the coil array controller 72, electromagnetic fields are generated within the patient 34 in the area where the medical procedure is being performed, which is again sometimes referred to as patient space. The electromagnetic fields generated in the patient space induce currents in the tracking device 84 positioned on or in the suction instrument 100. These induced signals from the tracking device 84 can be delivered to the navigation probe interface 80 and subsequently forwarded to the processor 32. The navigation probe interface 80 can also include amplifiers, filters and buffers to directly interface with the tracking device 84 in the instrument 100. Alternatively, the tracking device 84, or any other appropriate portion, may employ a wireless communications channel, such as that disclosed in U.S. Patent No. 6,474,341, entitled "Surgical Communication Power System," issued November 5, 2002, as opposed to being coupled directly to the navigation probe interface 80.

The tracking system 60, if it is using an electromagnetic tracking assembly, essentially works by positioning the coil arrays 64, 68 adjacent to the patient 32 to generate an electromagnetic field, which can be low energy, and generally referred to as a navigation field. Because every point in the navigation field or patient space is associated with a unique field strength and directions, the electromagnetic tracking system 60 can determine the position of the instrument 100 by measuring the field strength and directions or components thereof at the tracking device 84 location. The coil array controller 72 can receive the induced signals from the tracking device 84 and transmit information regarding a location, where location information can include both x, y, and z position and roll, pitch, and yaw orientation information, of the tracking device 84 associated with the tracked suction instrument 100. Accordingly, six degree of freedom (6 DOF) information can be determined with the navigation system 10.

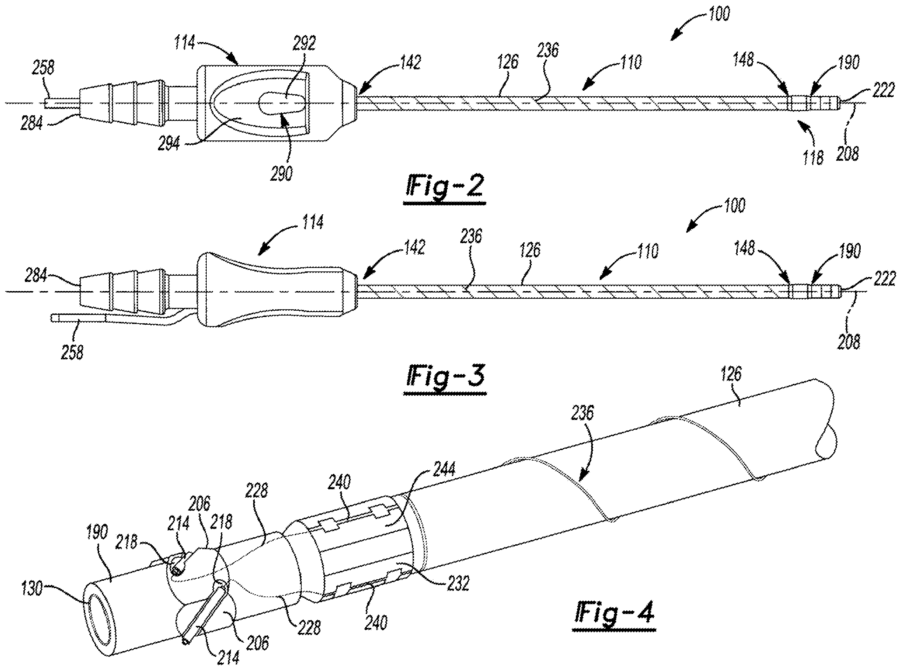

Referring now to Figures 2-10, the navigated malleable surgical instrument 100 will be described in greater detail. In one exemplary configuration, the malleable surgical instrument 100 can be used for suction, including fluid and tissue removal in ENT procedures. It should be appreciated, however, that the navigated malleable surgical instrument 100 can be used in various other surgical procedures as may be desired and can be provided in the form of a malleable or flexible endoscope, a malleable or flexible catheter, and/or a malleable cannula. Thus, while the following description continues with reference to a navigated malleable suction instrument 100, the discussion is also applicable to the surgical instruments discussed above.

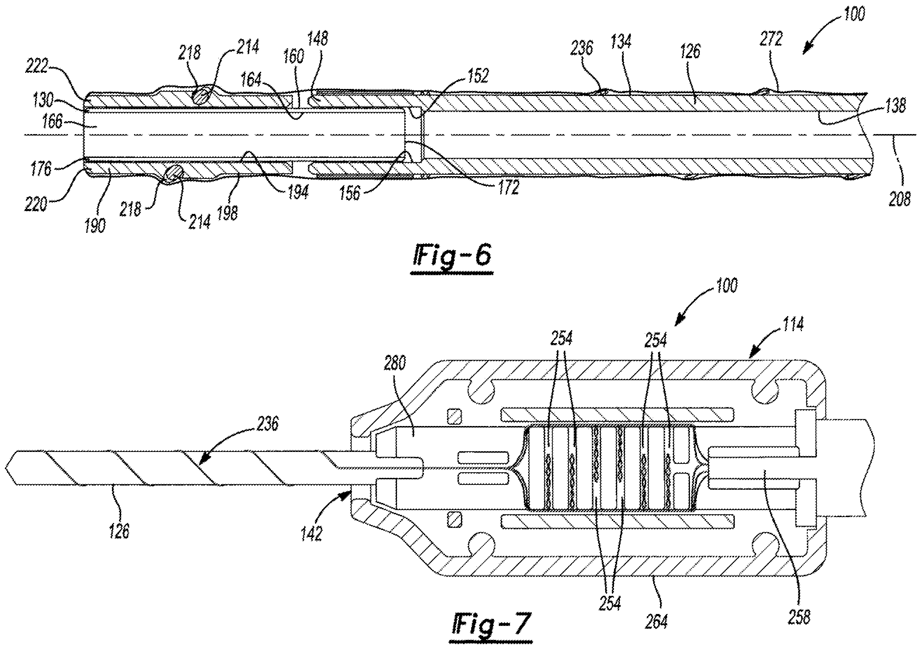

Suction instrument 100 can include a tube assembly 110, a handle assembly 114 and a tracking sensor arrangement 118. Suction instrument 100 can be configured for a single use such that it would be disposed after such use. The tube assembly 110 can include a malleable elongated tubular body 126 and an insert portion 130. The tubular body 126 can include an outer diameter 134 and an inner diameter 138 and can have a first end 142 coupled to the handle assembly 114 and a second opposite end 148 configured to receive insert portion 130, as shown in Figure 6. The second end 148 can include an internal annular recess 152 having an inner diameter 156 greater than the inner diameter 138 of the remaining portion of body 126, as also shown in Figure 6. The malleable elongated body 126 can be formed from various aluminum alloys, such as AL 3003-0, various stainless steel alloys, such as 304 annealed, as well as various other materials including titanium, niobium, molybdenum, tantalum, nitinol, vinyl, and multi-lumen materials, such that it is malleable to facilitate being bent or formed into various configurations and retaining the bent or formed configuration, as will be discussed herein. The body 126 can also be provided in various lengths and diameters, including 7, 9 and 12 French diameters (2.3, 3.0 and 4.0 mm).

The insert portion 130 can be configured to provide non-malleable support for at least the tracking sensor 84. Insert portion 130 can include an outer diameter 160 substantially equal to the inner diameter 156 of annular recess 152, and an inner diameter 164 substantially equal to the inner diameter 138 of malleable elongated body 126, as also shown in Figure 6. In this manner, the substantially equal inner diameters 138, 164 can provide for a substantially constant flow path 166 for suction. It should be appreciated, however, that the inner diameters 138, 164 can also be provided with varying dimensions. The insert portion 130 can also include an exemplary axial length of 10 to 15 mm, including 14 mm. Insert portion 130 can include a first end 172 and a second opposite end 176. The first end 172 of the insert portion 130 can be received in annular recess 152, as shown in Figure 6. Insert portion can include a rigid construction to facilitate receiving and housing tracking device 84, as will be described herein. In this manner, insert portion 130 can be formed or manufactured from stainless steel or other biocompatible rigid materials such that insert portion 130 is not malleable like elongated body 126. The insert portion can also include an exemplary axial length of approximately 10 mm.

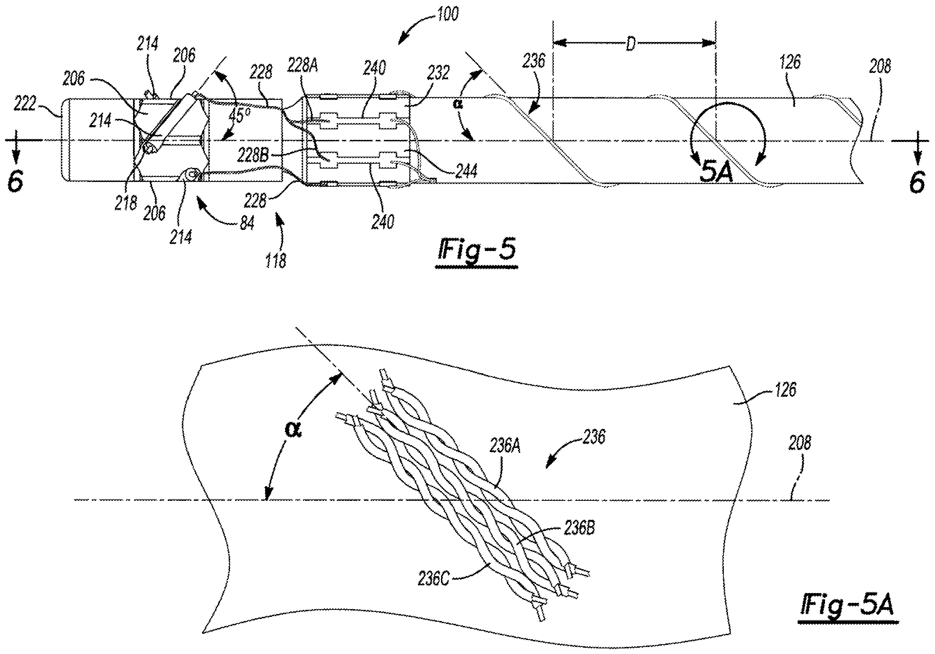

Insert portion 130 can include a sleeve 190 received on an exterior thereof, as shown in Figures 5 and 6. Sleeve 190 can include an inner diameter 194 substantially equal to the outer diameter of insert portion 130, and an outer diameter 198 substantially equal to the outer diameter 134 of body 126. It should be appreciated that sleeve 190 can also be configured with different diameters relative to body 126. Sleeve 190 can extend over a portion of insert 130 from the first end 172 of the insert portion 130 towards the second end, as shown in Figure 6. In one exemplary configuration, sleeve 190 can extend from the first end 172 and contact the first end 142 of body 126 when the insert portion 130 is coupled to annular recess 152 of body 126. In another exemplary configuration, sleeve 190 can extend from the first end 172 of body portion 130 in a similar manner as discussed above, but can stop short of the first end 142 of body 126, as shown in Figure 6. Sleeve 190 can be fixed to insert portion 130, and insert portion 130 can be fixed to annular recess 152 with an appropriate adhesive. Sleeve 190 can be formed of a polymeric material or other suitable materials. Sleeve 190 can also include a first end 220 configured to substantially align with the second end 176 of insert 130. The first end 220 can include a rounded or chamfered blunt distal tip or end part 222 such that it can be placed against surrounding tissue during a suction procedure without cutting or damaging such tissue. In one exemplary configuration, end part 222 can extend over insert portion 130 so as to prevent cutting or damaging tissue.

With particular reference to Figures 4 and 5, sleeve 190 can include a plurality of flattened sections 206 configured to facilitate receiving and supporting the tracking sensor arrangement 118, as will be described herein. In one exemplary configuration, sleeve 190 can include at least three flattened sections 206 configured to attachably receive tracking device 84. In this configuration, the tracking device 84 can include three coil assemblies 214, as will be described herein. Briefly, in one exemplary configuration, the three coil assemblies 214 can each include a cylindrical configuration as shown in Figures 4 and 5, having an overall axial length of approximately 1.5 mm to 2.7 mm, an overall diameter of approximately 0.3 to 0.6 mm, and a plurality of wire windings wound along a cylindrical base to form the cylindrical configuration. The plurality of windings can form the coil assembly 214 having the generally uniform cylindrical configuration, as generally shown in Figure 5. Each flattened section 206 can include a slot or depression 218 formed therein and configured to receive a corresponding coil assembly 214, as shown for example in Figures 5 and 6. Each slot 218 can be formed in the corresponding flattened section 206 at a 35 to 75 degree angle, including a 55 degree angle, to a longitudinal axis 208 of the tube assembly 110. In one exemplary configuration, each slot 218 can be formed at a 55 degree angle to longitudinal axis 208, as shown in Figure 5. Each of the three flattened sections 206 can be positioned equidistantly or 120 degrees around a circumference of sleeve 190 so that the three coil assemblies 214 are therefore likewise positioned equidistantly around the circumference of sleeve 90, as also generally shown in Figures 4-6. It should be appreciated that the coil assemblies can also be coupled to the sleeve without the flattened sections 206, and can be aligned at different orientations relative to the longitudinal axis, including parallel thereto. In this regard, the sleeve 190 can include an outer surface with a circular shape in cross-section configured to receive the coil assemblies 214.

The coil assemblies 214 can include three coil assemblies as described above that cooperate with the navigation system 10 such that 6 DOF tracking information can be determined. It should be appreciated, however, that two coil assemblies 214 could also be used in conjunction with navigation system 10 such that 6 DOF tracking information can also be determined. In a configuration where three coil assemblies 214 are utilized, two of the three coil assemblies can be positioned at an angle relative to the longitudinal axis 208 with the third coil assembly being positioned at an angle relative to the longitudinal axis 208 or parallel thereto. The three coil assemblies 214 can also each be positioned at an angle relative to each other. As discussed above, an exemplary angle of the three coil assemblies 214 relative to the longitudinal axis 208 can be 55 degrees, which also provides for optimal packaging and spacing of the coil assemblies circumferentially around sleeve 190. It should be appreciated that while an angle of 55 degrees has been discussed, other angles could be utilized with coil assemblies 214 and instrument 100 as may be required. It should also be appreciated, as discussed above, that the coil assemblies could be positioned parallel or perpendicular to the longitudinal axis 208.

In a configuration where tracking device 84 includes two coil assemblies 214, the two coil assemblies can similarly be positioned equidistant or 180 degrees spaced around an outer perimeter of sleeve 190, as well as can each be positioned at an angle relative to each other and at an angle relative to the longitudinal axis 208 of the tube assembly 110. In this configuration, the two coil assemblies can also cooperate with navigation system 10 such that 6 DOF tracking information can be determined. In one exemplary configuration, the two coil assemblies 214 can be positioned at an angle of about 35 to 75 degrees, including about 55 degrees relative to longitudinal axis 208 of the tube assembly 210.

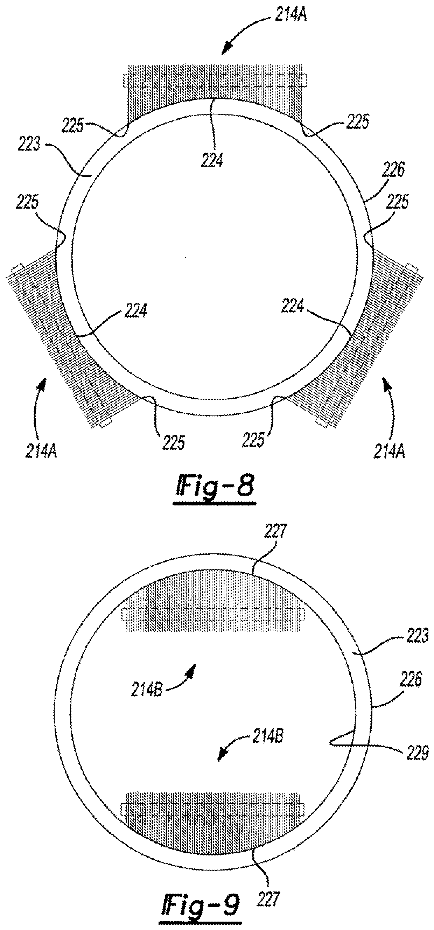

With additional reference to Figures 8 and 9, two exemplary coil assemblies 214A and 214B having alternative winding configurations are illustrated operatively associated with an exemplary tubular structure 223 of an exemplary instrument. Coil assemblies 214A and 214B can each include an overall non-linear shape as compared to the overall cylindrical configuration of coils assemblies 214 shown in Figure 5. Coil assembly 214A can include a central arcuate depression or concavity 224 such that the depression 224 has a smaller outer diameter than opposed ends 225 of the plurality of windings, as generally shown in Figure 8. The winding configuration of coil assembly 214A can provide an ability to maximize an amount of coil windings on a base wire while working towards minimizing an overall outer dimension or size of an instrument. In this regard, coil assembly 214A is shown in Figure 8 with the arcuate depression 224 substantially conforming to an outer surface 226 of the tubular structure 223 such that the coil assembly or assemblies 214A essentially nest around the outer surface 226 of the tubular structure. In this regard, because of the general clearance provided by a cylindrical coil assembly positioned adjacent to an outer diameter of the tubular structure 223, a gap or space 221 on either end of the coil can include additional windings without effectively increasing the overall outer diameter of the entire assembly. This can allow for greater or stronger sensitivity in the navigated space.

With particular reference to Figure 9, coil assembly 214B can include an overall arcuate convex shape 227 configured to conform to and nest within an inner diameter 229 of the exemplary tubular structure. Similar to coil assembly 214A, such a configuration can provide for maximizing an amount of windings on the base wire while also working towards minimizing the inner diameter 229 of the tubular structure 223 that would be required to receive one or more coil assemblies 214B.

With particular reference to Figures 5 and 5A, the tracking sensor arrangement 118 will now be described in detail. Tracking sensor arrangement 118 can include the tracking device 84 having the two or three coil assemblies 214, as well as a first set of lead wires 228, the flexible printed circuit board or sheet 232 and a second set of lead wires 236. The first set of lead wires 228 can include a pair of lead wires 228A and 228B for each coil assembly 214, as generally shown in Figure 5. Each respective pair of lead wires 228A and 228B can be routed to a first end of a respective pair of circuit connections 240 on flexible printed circuit sheet 232. As will be discussed in greater detail below, the flexible circuit sheet 232 can facilitate improving the time and cost associating with terminating fine wires utilized in medical and other instruments while also providing the flexibility necessitated for such instruments. It should be appreciated that while tracking device 84 is described as having three coil assemblies, more or less coil assemblies can be utilized as may be desired or required depending on, for example, characteristics of the navigation system being utilized as well as the number of degrees of freedom desired.

The flexible printed circuit sheet 232 can include a flexible backing or base layer 244 such that it can readily conform to the contour of an outer surface of the body 126, as shown for example in Figure 4. The flexible printed circuit sheet 232 can wrap entirely or partially around a perimeter of the body 126 and can be positioned adjacent the second end 148 of body 126, as generally shown in Figures 5 and 6. In this manner, the insert portion 130, in its inserted position shown in Figure 6, can be under all or substantially all of the flexible printed circuit sheet 232. The rigid insert portion 130 can thus prevent the malleable body 126 from bending or flexing in a region of the flexible printed circuit sheet 232. In one exemplary configuration, the flexible printed circuit sheet 232 can be an integral part of sleeve 190. In another exemplary configuration, flexible printed circuit sheet 232 can be positioned in a similar manner on sleeve 190. In this configuration, flexible printed circuit sheet 232 can be positioned on sleeve 190 between coil assemblies 214 and the end of sleeve 190 adjacent the second end 148 of body 126.

The second set of lead wires can include three respective pairs of wires 236A, 236B, 236C, as generally shown in Figure 5 with reference to the partial exploded view in Figure 5A. It should be appreciated that while Figures 2-5, 6-7 and 10 show the second set of lead wires 236 as one element, this is for illustration purposes only and it should be understood that the second set of lead wires shown in Figures 2-5, 6-7 and 10 include the three respective pairs of lead wires 236A-C, as shown in Figure 5A. Each pair of lead wires 236A-C can be twisted together and positioned adjacent each other, as also shown in Figure 5A. The twisted pairs 236A-C of wires can reduce electrical interference or cross-talk between each pair of adjacent lead wires as well as minimize pickup from an associated electromagnetic navigation system. Each pair of lead wires can be connected to a single coil assembly 214 via the flexible printed circuit sheet 232. The lead wires can also include a Teflon coating or other appropriate lubricous or friction reducing coating on an outer surface thereof. Each pair of lead wires 236A-C can be coupled to an opposite end of respective circuit pads 240 on the flexible printed circuit sheet 232. It should be appreciated that the lead wires 228 could alternatively extend up the body 126 as a twisted pair of lead wires without the use of the flexible printed circuit sheet 232, or could extend up to and be terminated directly to the respective twisted pair of lead wires 236.

The second set of lead wires 236, which includes the three pairs of twisted wires 236A-C, can be helically wound around elongated body 126 from the flexible printed circuit sheet 232 to the second end 148, as generally shown for example in Figures 3-5A. The wires 236 can be wound around the outside of body 126 at an angle α relative to the longitudinal axis 208 of approximately 0 to 85 degrees, including about 30 degrees, as generally shown in Figures 5 and 5A. Each revolution of the wires 236 around body 126 can be spaced apart from each other by a distance D of approximately 2 to 45 mm, including about 5 mm, as shown with reference to Figure 5. In one exemplary configuration, the range can include from about 15 - 45 mm. The helical winding of the wires 236 at an acute angle relative to the longitudinal axis along with the relatively close spacing of the wires and the Teflon coating facilitate being able to bend the malleable body 126 at significant angles, including beyond ninety degrees, without breaking or otherwise damaging the wires 236, as will be discussed herein. It should be appreciated that the wires 236 can also be positioned along body 126 in a single revolution from the flexible printed circuit sheet 232 or the tracking device 84 to the second end 148. In this regard, the revolution spacing can be from about 2 mm to a length of the body 126. The wires 236 can also be positioned along body 126 from the flexible printed circuit sheet 232 to the second end 148 without being wound around body 126.

Once the second set of wires 236 has been helically wound around the outside of tubular body 126 to the first end 142, the wires can be routed into slots 254 in handle assembly 114 and connected to respective lead wires of a cable connector assembly 258, as generally shown in Figure 7. The cable connector assembly 258 can be connected to the navigation probe interface 80, as generally shown in Figure 1. The handle assemble 114 can include two half sections 264, with one half section being shown in Figure 7 for illustration purposes.

With particular reference to Figure 6 and continued reference to Figures 2-5A, 7 and 10, the tube assembly 110 can include a polymeric outer heat shrink 272 covering the entire assembly, as shown in the cross-sectional view of Figure 6. Thus, the heat shrink 272 can cover the elongated body 126, the insert portion 130, and the sensor arrangement 118 including the wires helically wound along the body 126. The heat shrink 272 can provide an outer covering or shell over the tube assembly 110 and sensor arrangement 118 while providing sufficient flexibility for both bending of the body 126 and slight relative movement of the helically wound wires 236 as a result of the bending. In this regard, the wires can be moveably captured between the heat shrink and the tubular body. The heat shrink covering can also serve as an electric isolation barrier. It should be appreciated that while the heat shrink covering is only shown in Figure 6, it has not been shown in the other various views for clarification purposes only to better illustrate the sensor arrangement 118 and routing of wires 236. In this regard, it should be understood that the heat shrink 272 can cover the tube assembly 110 and sensor arrangement 118 shown in Figures 2-10.

As discussed above, the handle assembly 114 can include multiple components, such as for example two halves, with one of the halves shown in Figure 7 receiving the first end of the suction tube assembly 110 in fluid communication with a suction passage 280 formed therein. The suction passage 280 can terminate at a connector 284 protruding from a proximal end of the handle (Figures 2 and 3) and can be configured to receive a suction hose or other arrangement in fluid communication with a suction source (not shown). Once the wires are connected to the cable assembly and routed in the slots 254 as discussed above, the other half of handle assembly 114 can connected and an adhesive can be used to bond the handle halves together to form the handle as shown in Figures 2 and 3.

With particular reference to Figure 2, handle assembly 114 can include a suction adjustment feature 290 which can be in the form of a bore 292 extending from an outer surface 294 of the handle assembly 114 and into fluid communication with the suction passage 280. In operation, a surgeon or user 50 of the instrument 100 can place their thumb or another object over the bore 292 to vary an opening of the bore 292 and thus vary an amount of suction pressure realized in the flow path or passage 166. For example, if the bore 292 is left completely open or uncovered, a majority if not all of the suction will be through the bore 292 and not the first end 172 of insert portion 130. On the other hand, if the bore 192 is completely covered or closed off, a maximum amount of suction will be realized at end 172. Varying the opening of bore 292 between fully closed and fully opened can therefore correspondingly vary an amount of realized suction at end 172.

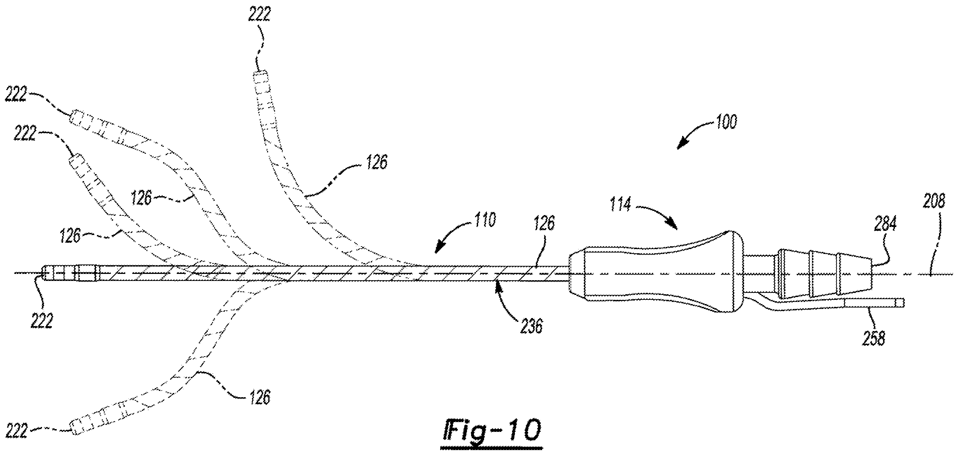

In operation and with additional reference to Figure 10, the malleable elongated body 126 can be bent into various configurations, as generally shown by the exemplary configurations 300A-D. The malleable nature of body 126 can provide the ability for body 126 to be bent into such various configurations without kinking and can maintain the various configurations until bent or shaped into another configuration. Further, malleable body 126 can be bent or shaped as discussed above without require additional tools, such as a mandrel to facilitate the bending. This is advantageous, for example, in that a surgeon can bend body 126 multiple times by hand during a procedure in close proximity to the patient without having to resort to additional tools or other equipment to facilitate the bending while performing the procedure.

Moreover, the helically wound configuration of wires 236 along with the Teflon coating provides for the ability to bend malleable body 126 at various angles including through ninety degrees without breaking the wires. More specifically, by winding wires 236 helically around body 126 at an angle relative to the longitudinal axis and at a close proximity to each other, the wound wires can conform to the bent shape and move or flex axially with the bent tube such that they do not strain and/or break during the bending. In addition, the Teflon coating provides added lubricity for the wires to have relative motion between the tube and the outer shrink coating 272 during bending.

Further, by providing the tracking device 84 near the distal tip 222, the distal tip 222 of the suction instrument can be tracked to provide substantially accurate position data for the distal tip of suction instrument 100 when out of a line of sight in a body cavity of patient 34. This is particularly useful for the malleable suction instrument 100 because, for example, the tip can be bent or moved relative to the handle and still be tracked. On the other hand, if the tracking device was in the handle (such as in a hind tracked system) and the body 126 was subsequently bent or shaped, the navigation system would no longer be able to accurately track the position of the distal tip. In this regard, the present teaching provide a tip tracked malleable suction instrument that can be bent or shaped into various configurations as may be required during a procedure, and the distal tip can be accurately tracked in any of the various bent positions.

In use, the patient 34 can be positioned on an operating table or other appropriate structure and appropriate image data of a patient or navigation space can be obtained, such as an ENT area. The image data can be registered to the navigation space as is known in the art. The surgeon 50 can determine a shape of the malleable suction instrument 100 to reach a target site and bend the suction instrument 100 to the determined shape where instrument 100 retains the bent shape, as discussed above. The bent or shaped surgical instrument 100 can then be guided to the target site with crosshairs representing the position of the distal tip of instrument 100 being superimposed on the image data. The crosshairs can show the tracked relative position of the distal tip as instrument 100 is navigated to the target site. In addition, if during navigation of the shaped instrument 100 to the target site, the surgeon determines that the shaped configuration will need to be altered, the surgeon can bend and/or reshape the instrument 100 to a newly shaped configuration and proceed again as discussed above.

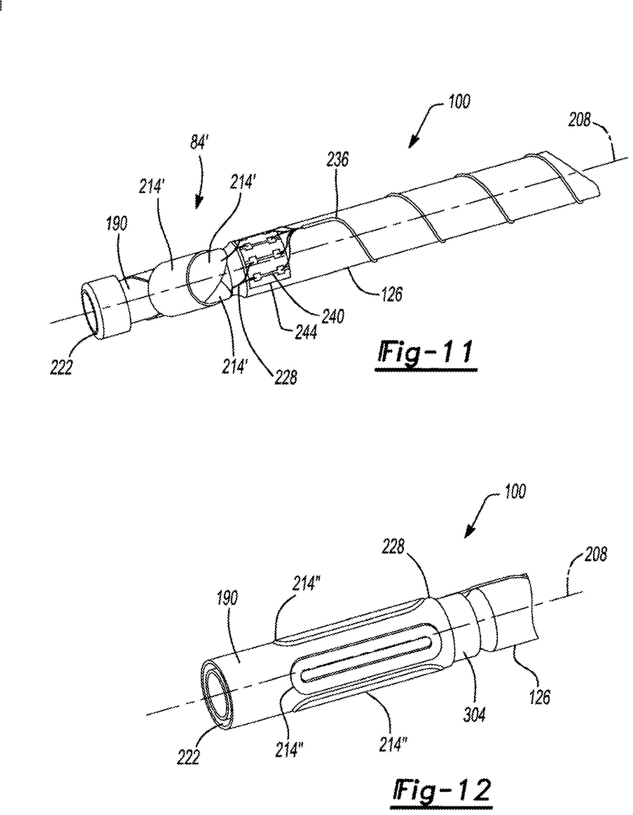

With additional reference to Figure 11, an alternative tracking device arrangement 84' will now be discussed. As can be seen in Figure 11, tracking device 84' can include two or three wrapped coil assemblies 214' that can be used in place of the coil assemblies 214. Coil assemblies 214' can be wrapped around sleeve 190 proximate the distal tip 222. In one exemplary configuration, the coil assemblies 214' can be individually wrapped around sleeve 190 in an overlapping manner with a wrap axis having a non-normal and non-parallel angle to longitudinal axis 208. In the exemplary configuration illustrated, coil assemblies 214' can be wrapped around sleeve 190 at an angle relative to each other and longitudinal axis 208. In another exemplary configuration, coil assemblies 214' can be wrapped around sleeve 190 and spaced axially apart from each other. A further discussion of the coil assemblies 214' can be found in U.S. Application Serial No. 12/770,181, filed on April 29, 2010 and entitled "Method and Apparatus for Surgical Navigation".

With additional reference to Figure 12, another alternative tracking device arrangement 84" is shown associated with instrument 100. Tracking device 84" can also be used in place of tracking device 84 and can include a plurality of oval coil assemblies 214" positioned about sleeve 190 proximate distal tip 222. In one exemplary configuration, two to four coil assemblies 214" can be positioned about sleeve 190 proximate distal tip 222. In the exemplary configuration illustrated, four coil assemblies 214" can be circumferentially spaced around sleeve 190 proximate distal tip 222, and an axial coil 304 can be positioned proximally of coil assemblies 214", as shown in Figure 12. In one exemplary configuration, two oval coil assemblies 214" can be provided with the axial coil 304. The two coil assemblies 214" can also include two pair of coil assemblies 214" provided with the axial coil 304.

The coil assemblies 214" can be formed in various selected shapes, such as elliptical, circular, or oval. In one exemplary configuration, the axial coil 304 can be concentric with and wrapped around an outer surface of sleeve 190 or body 126, as shown in Figure 12. A further discussion of coil assemblies 214" and axial coil 304 can be found in U.S. Application Serial No. 13/016,740, filed on January 28, 2011 and entitled "Method and Apparatus for Image-Based Navgation".

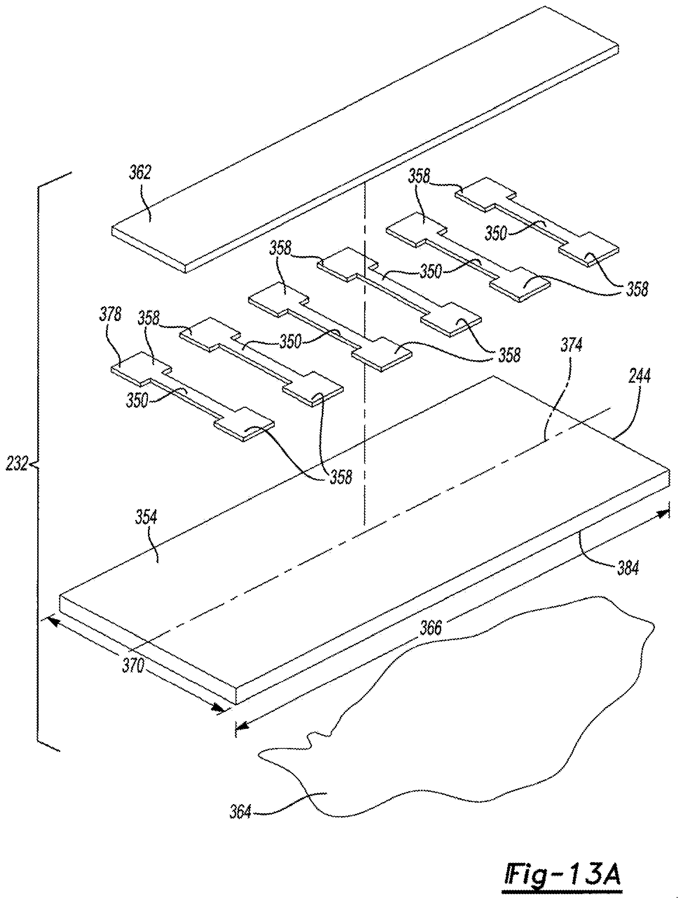

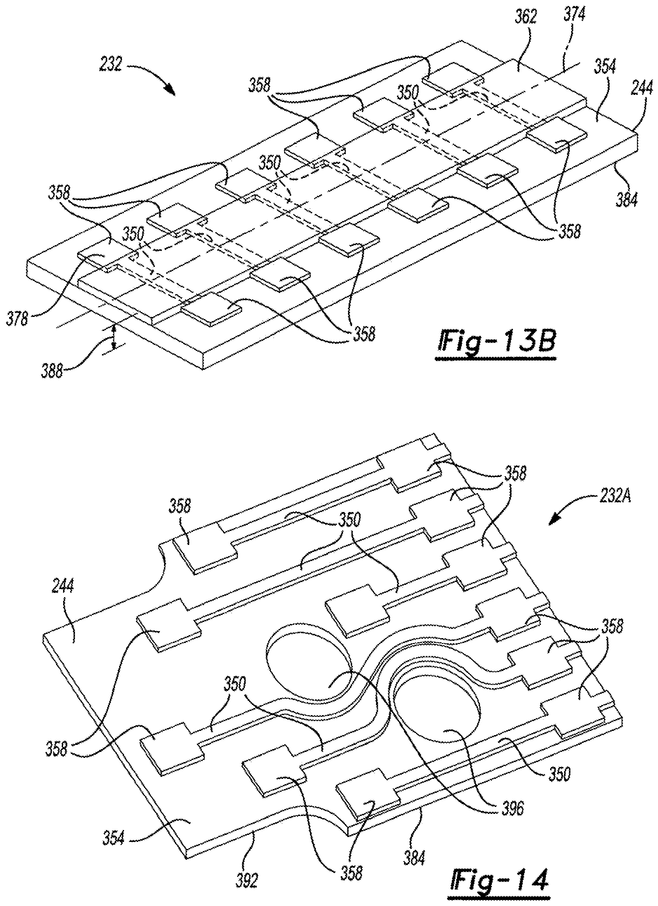

Turning now to Figures 13A-18, the flexible printed circuit sheet 232, including various exemplary configurations thereof, will now be discussed in greater detail. With particular reference to Figures 13A-13B, one exemplary configuration of the flexible printed circuit sheet 232 is shown in both an exploded view (Figure 13A) and an assembled view (Figure 13B). Flexible printed circuit sheet 232 can include the flexible backing or base layer 244, one or more circuit or conductive traces, such as copper traces 350, positioned on a first or upper side 354 of base layer 244, coupling pads 358 associated with traces 350, and an insulative layer 362 formed over at least the copper traces 350 and coupled to base layer 244. It will be appreciated that while copper traces 350 are shown positioned on upper side 354, the copper traces 350 can also be positioned on an opposite lower side of base layer 244. While the discussion will continue with reference to the conductive traces being copper traces 350, the conductive traces can also be formed from metal, nickel, gold, or copper with nickel/gold plating.

The flexible printed circuit sheet 232 can provide a mechanism for facilitating fine gauge wire termination of associated sensors or coils and lead wires, such as wires 228 and 236 of exemplary suction instrument 100. The flexible printed circuit sheet 232 can also enable manufacturing and design flexibility in connection with use of circuit sheet 232 on instruments and other devices that are flexible and/or conformable. For example, conventional techniques for electrically terminating sensor wires to lead wires can include directly connecting the sensor wires to the lead wires via soldering. As can be appreciated, such a technique is very time and labor intensive considering that the sensor and lead wires can include 58 AWG wire with an outer diameter of approximately 0.01 mm. Indeed, such conventional techniques for soldering the sensor wires to the lead wires often require performing the process under a microscope or other magnifying apparatus, which can further drive cost and expense into the manufacturing process.

As will also be discussed in greater detail below, the exemplary flexible circuit sheets discussed herein can provide for improved efficiency and cost reduction in terminating such fine gauge sensor and lead wires, especially for medical instruments having size or volume constraints and that also require flexibility or conformability. In this regard, the coupling 358 on the flexible circuit sheet 232 can be orders of magnitude bigger than the outer diameter of the wires to be terminated, such as a 0.1 mm to 0.5 mm square pad, for example. In an exemplary configuration, the coupling pads 358 can have a large surface area for the wires to be terminated such that, for example, a primary linear dimension of the coupling pads 358 can be orders of magnitude bigger than the outer diameter of the wires to be terminated. In one exemplary configuration, the wires to be terminated can include an outer diameter of between approximately 0.03 mm to 0.05 mm. In an exemplary configuration, the wires to be terminated can include 58 AWG wire having an outer diameter of approximately 0.01 mm. This can, among other things, facilitate easier and more efficient termination of the fine gauge wire due to the larger size of coupling pads 358.

The base layer 244 can be formed form various materials having appropriate insulative properties and appropriate material properties such that base layer 244 is flexible and can conform to various surface geometries. For example, the base layer 244 (as well as the assembled printed circuit sheet 232) can conform to the outer tubular surface of the malleable suction instrument 100. In one exemplary configuration, the flexible nature of flexible circuit sheet 232 can facilitate movement with tube assembly 110 of malleable instrument 100 (e.g., Figure 10) once adhered thereto. In one exemplary configuration, the base layer 244 can be formed from a polymeric material, including but not limited to, a polyimide. In the exemplary configuration illustrated in Figures 13A-13B, the base layer 244 can include a length 366 of approximately 7 mm and a width 370 of approximately 3 mm. It should be appreciated, however, that the size and shape of base layer 244 can vary depending on a particular application.

The copper traces 350 can be positioned or printed in any desired orientation on base layer 244, including substantially perpendicular to a longitudinal axis 374 of base layer 244. The copper traces 350 can similarly include varying lengths and widths depending on the particular configuration of flexible printed circuit sheet 232. In the exemplary configuration illustrated in Figures 13A-13B, the copper traces 350 can include a length of approximately 1.25-3.0 mm and a width of approximately 0.15 mm. The copper traces 350 can include a thickness of approximately 0.01 - 0.04 mm.

The coupling pads 358 can be positioned or printed at ends of the copper traces 350, as can be seen in Figures 13A-13B. The coupling pads 358 can be formed in any desired shape, including the square or substantially square shape 378 shown, for example, in Figures 13A-13B. The coupling pads 358 can also be formed to have varying dimensions, including a dimension or dimensions that is/are larger than a typical outer diameter of the wires that are to be coupled to the pads. As discussed above, such a greater dimension of the coupling pads 358 relative to the size of the wire can provide for easier soldering of the wires to the pads 358 and thus reduce time and manufacturing complexity associated with building an instrument requiring termination of fine gauge wires.

For example, the exemplary coupling pads 358 shown in Figures 13A-13B are square in shape and include a length and width of approximately 0.5 mm. Again, it should be appreciated that the length and width of coupling pads 358 can vary depending on the particular application of flexible printed circuit sheet 232. The coupling pads 358 can also be formed from copper and include a tinning material, such as tin/lead, nickel/gold and/or gold.

The insulative layer 362 can be positioned over the copper traces 350 and coupled to the base layer 244 in any suitable manner that allows or does not inhibit the flexibility and conformability of the flexible circuit sheet 232. In one exemplary configuration, the insulative layer 362 can be adhered to the base layer 244 and copper traces 350 with an adhesive. The insulative layer 362 can include a shape and/or width so as to cover or substantially cover the copper traces 350 between the coupling pads 358 to insulate the traces 350 from external contact. Similar to the base layer 244, the insulative layer 362 can also be formed from a polymeric material, such as polyimide. In one exemplary configuration, the insulative layer 362 can be a photoimageable coverlay. As will be discussed in greater detail below, the insulative layer 362 can include a thickness that is less than a thickness of the base layer 244. In the exemplary configuration shown in Figures 13A-13B, the insulative layer 362 can include a rectangular shape corresponding to the exemplary symmetrical positioning of the copper traces 350 and corresponding coupling pads 358.

To couple the flexible printed circuit sheet 232 to a structure, such as the exemplary instrument 100, an adhesive 364 can be used. It should be appreciated, however, that other means for securing the flexible circuit sheet 232 to a structure can be used, so long as the means used does not inhibit the flexible nature of printed circuit sheet 232. In one exemplary configuration, the adhesive 364 can be applied to a lower or second side 384 of base layer 244. In this regard, the second side of base layer 244 can be substantially smooth. It should also be appreciated that the adhesive 364 can also be applied to the structure in addition to or in lieu of being applied to base layer 244. In one exemplary configuration, the adhesive 364 can include a medical grade pressure sensitive adhesive. In another exemplary configuration, the adhesive 364 can include a medical grade liquid or gel adhesive.

The exemplary flexible printed circuit sheet 232, in the exemplary assembled configuration shown in Figure 13B, can include a bound together or overall thickness 388 of between approximately 0.04 - 0.07 mm. In some exemplary embodiments, the overall thickness 388 can be only approximately 0.04 mm. Stated another way, the assembled base layer 244, circuit traces and pads 350, 358 and insulative layer 263 can include an overall thickness 388 of approximately 0.05 mm. It should be appreciated, however, that such a thickness can vary to be smaller or larger depending on the particular application of the flexible printed circuit sheet 232. Use of the pressure sensitive adhesive 364 can increase the overall thickness 388 by approximately 0.025 mm to 0.05 mm. Similarly, use of the gel or liquid adhesive can increase the thickness 388 by only 0.01 mm. Thus, the overall thickness 388 of the flexible printed circuit sheet 232, in various different configurations, can vary from 0.04 mm (without adhesive 364) to approximately 0.11 mm (with adhesive 364). As discussed above, such a minimal thickness 388 of flexible circuit sheet 232 provides for not only flexibility and conformability of the circuit sheet 232, but also applicability of the flexible circuit sheets to medical and other devices and/or instruments that have very tight volume and/or packaging constraints.

For example, one of ordinary skill in the art will appreciate that conventional printed circuit boards considered thin in the industry can include a thickness of 0.8 mm or greater and can be made from dielectric layers laminated together with epoxy resin prepreg. Such materials combined with such a thickness do not provide for the conventional circuit boards being flexible and thus they cannot conform to non-planar surfaces and/or flex such that they cannot be used with a flexible or malleable medical instrument. Further, such a thickness of 0.8 mm or greater can preclude use of conventional printed circuit boards in medical instruments or devices where maintaining a minimum thickness or overall height is a critical parameter.

The very thin thickness 388 of the exemplary flexible circuit sheet 232, together with the polyimide material construction, can provide for significant flexibility and/or conformability of circuit sheet 232. In this regard, the exemplary flexible circuit sheet 232 having the overall thickness 388 and polyimide material construction can include a bend radius of approximately ten times the thickness 388. Thus, for the exemplary configuration of flexible circuit sheet 232 discussed herein, the bend radius can be approximately 0.4 mm to 0.7 mm depending on the overall thickness 388 of the flexible printed circuit sheet 232. Such a bend radius can provide for significant flexibility in conforming the flexible printed circuit sheet to or around tight radii associated with compact or low profile medical instruments or devices.

With additional reference to Figures 14-16, another exemplary flexible printed circuit sheet 232 will now be discussed and designated with reference numeral 232A. Flexible printed circuit sheet 232A can include similar properties and thickness dimensions as discussed above for flexible circuit sheet 232 such that like reference numerals refer to like features or components. Flexible printed circuit sheet 232A is shown having an exemplary custom shape 392 configured for a particular medical instrument or device. In the exemplary configuration illustrated in Figures 14-16, flexible printed circuit sheet 232A can include one or more apertures 396 configured to be positioned around and/or provide access to corresponding coil assemblies 214. The copper circuit traces 350 can be printed in various patterns to accommodate the apertures 396 and custom shape 392, as shown for example in Figure 14. It should be appreciated that while not shown for clarity purposes, the insulative layer 362 can be custom shaped to include appropriate cutouts and an appropriate shape to cover the copper traces 350 while leaving the coupling pads 358 of flexible circuit sheet 232A exposed.

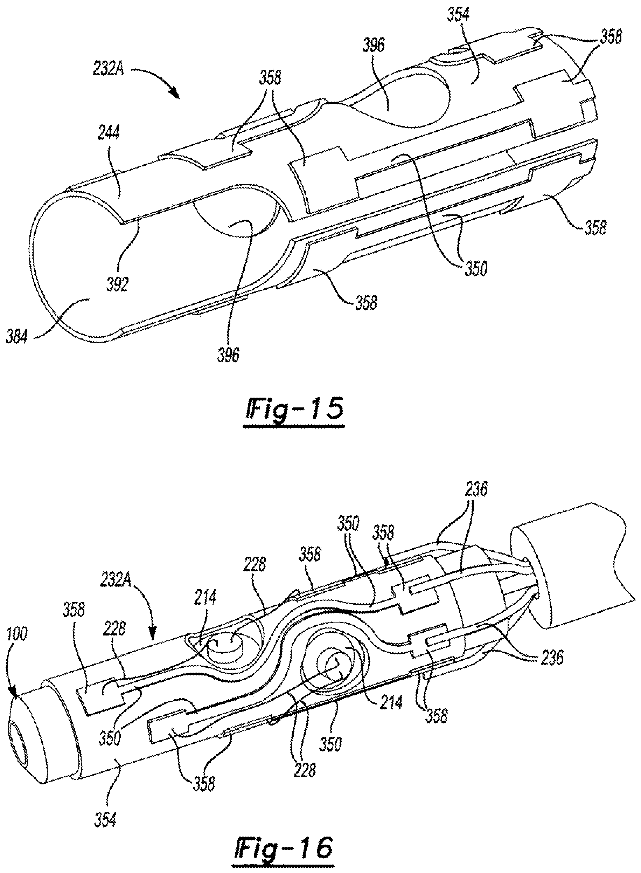

As can be seen in Figures 15-16, the flexible printed circuit sheet 232A can be bent or flexed in various configurations to conform to various instrument or device shapes, such as the distal end of a malleable instrument 100. In one exemplary configuration, the flexible printed circuit sheet 232A can wrap around or substantially around the malleable suction instrument 100. The flexible printed circuit sheet 232A can also bend, flex or twist with the malleable suction instrument 100 during use thereof. In this regard, the flexible printed circuit sheet 232A can flex three-dimensionally. In the exemplary configuration shown in Figure 16, flexible circuit sheet 232A can be adhered to the outer surface of a component of malleable suction instrument 100 using, for example, adhesive 364. As discussed above, the lead wires 236A can be electrically coupled, such as via soldering, to the appropriate coupling pads 358 and the coil assembly wires 228 can be soldered to the corresponding pads 358, as also shown in Figure 16.

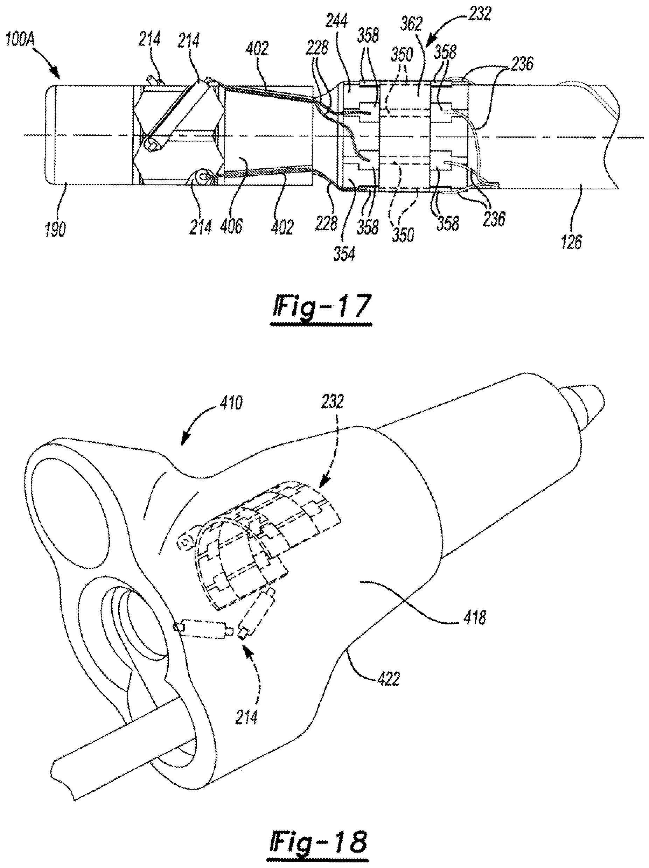

Turning now to Figure 17, flexible printed circuit sheet 232 is shown adhered to malleable suction instrument 100A, which is substantially similar to malleable suction instrument 100 shown in Figure 5, except for channels 402 formed in sleeve 190. Channels 402 can receive sensor or coil wires 228 and provide a predetermined routing placement for wires 228 relative to instrument 100A, as well as position wires 228 below an outer surface 406 of sleeve 190. Flexible printed circuit sheet 232 can conform to an outer surface of malleable suction instrument 100A and can provide for efficient and cost effective termination of coil assembly wires 228 and lead wires 236, as shown for example in Figure 17. For example, flexible circuit sheet 232 can be flexed to correspond to a radius of the outer surface of the instrument so as to lay substantially flush or coplanar to the outer surface.

With particular reference to Figure 18, flexible printed circuit sheet 232 is shown associated with an electromagnetic patient tracker device 410. In the exemplary configuration illustrated, tracker device 410 can include the three coil assemblies 214 positioned equidistant circumferentially around a longitudinal axis 414 of tracker device 410 and can be configured to communicate with and be tracked by EM tracking system 60 of navigation system 10. The coil assemblies 214 can also be positioned, in the exemplary configuration illustrated, at an angle, such as between forty-five degrees and fifty-five degrees relative to axis 414 in a similar manner as coil assemblies 214 are positioned relative to instrument 100 shown in Figure 5. It will be appreciated, however, that various other coil assembly 214 configurations and/or orientations can be utilized with patient tracker 410.

The flexible printed circuit sheet 232 can be positioned inside of or within a body 418 of tracker 410 as shown in Figure 18, or could alternatively be positioned on an outer surface 422 of tracker 410. In one exemplary configuration, flexible printed circuit sheet 232 can be bent or flexed to conform to the shape or contour of the surface it will be adhered to, as shown in Figure 18. Sensor and lead wires (not shown for clarity) can be soldered to the respective circuit pads in the manner discussed above.

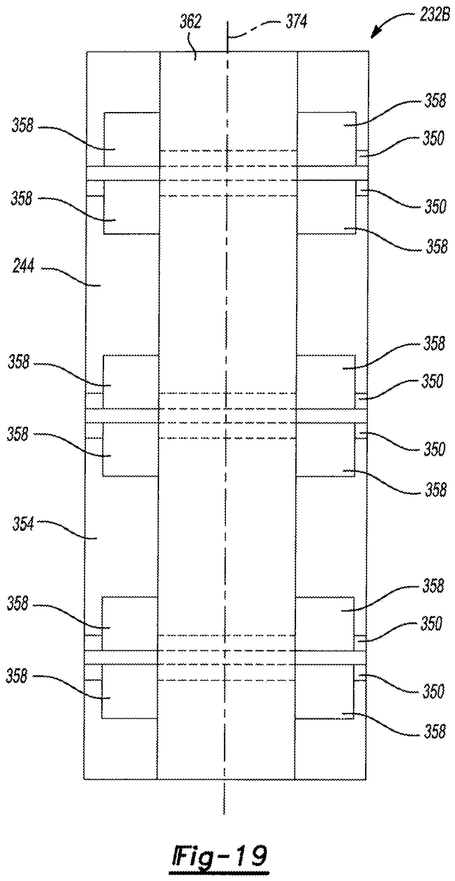

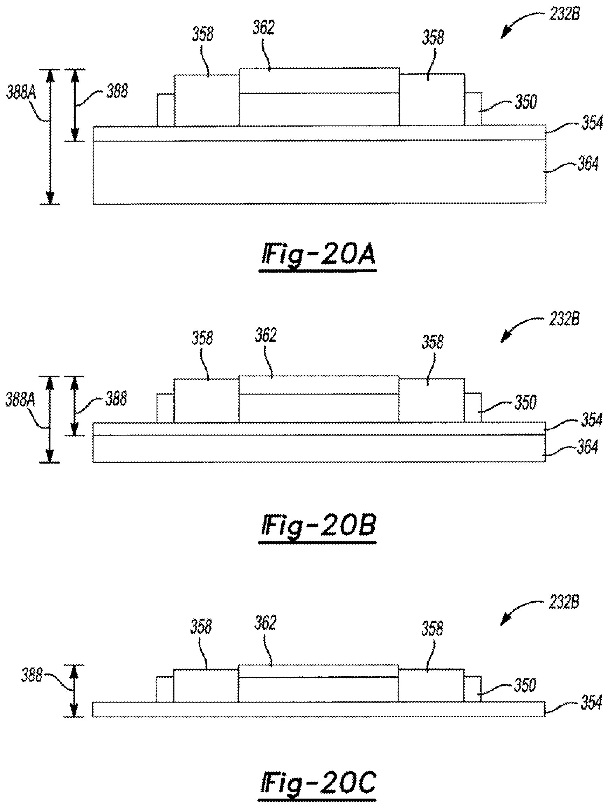

Turning now to Figures 19 and 20A-20C, another exemplary configuration of a flexible printed circuit sheet is shown at 232B. Flexible printed circuit sheet 232B can be similar to flexible printed circuit sheet 232A such that like reference numerals refer to like components or features and only differences will be discussed in detail. Similar to flexible printed circuit sheet 232A, the flexible printed circuit sheet 232B can include base layer 244 having upper surface 354, conductive traces 350, solder or coupling pads 358 and top insulative layer 362.

The flexible printed circuit sheet 232B can include one or more paired circuit traces where the pairs of circuit traces are closely spaced together, as shown for example in Figure 19. By positioning the circuit traces in such a manner along the longitudinal axis 374, any electromagnetic interference and/or pickup from an associated electromagnetic navigation system can be minimized. In this exemplary configuration, the conductive traces 350 can be parallel or substantially parallel to each other and spaced apart by less than 0.3 mm, including 0.23 mm, in each pair of circuit traces. However, it should be appreciated that other spacing may be utilized depending on design and other variables.

With particular reference to Figures 20A-20C, three exemplary configurations (shown in side views) of the flexible printed circuit sheet 232B are shown. In these exemplary configurations, various different thicknesses of the flexible printed circuit sheet 232B are shown with and without adhesive, as will be discussed in greater detail below.

Referring to Figure 20A, flexible printed circuit sheet 232B is shown in a configuration utilizing adhesive 364. In this configuration, the base layer 244 can include a thickness of approximately 0.01 mm, the conductive traces and pads 350, 358 can include a thickness of approximately 0.04 mm and the insulative layer 362 can include a thickness of approximately 0.02 mm. In the assembled configuration, the flexible printed circuit sheet 232B shown in Figure 20A can include an overall thickness 388 of approximately 0.07 mm without adhesive 364 and an overall thickness 388A of 0.11 mm with adhesive 364.

With reference to Figure 20B, the flexible printed circuit sheet 232B is shown having a smaller overall thickness 388 of approximately 0.05 mm without adhesive 364 and an overall thickness 388A of 0.07 mm with adhesive 364. In this configuration, the base layer 244 can similarly have a thickness of approximately 0.01 mm, the conductive traces and pads 350, 358 can include a thickness of approximately 0.02 - 0.03 mm and the insulative layer can include a thickness of approximately 0.01 mm.

Referring now to Figure 20C, the flexible printed circuit sheet 232B is shown in another exemplary configuration having an overall thickness 388 of approximately 0.04 mm. In this configuration, adhesive 364 may not be utilized. In such a configuration where adhesive 364 is not utilized, a heat shrink layer over the flexible printed circuit sheet 232B can optionally be utilized to couple flexible printed circuit sheet 232B to an instrument, such as the suction instrument 100 discussed above. In this configuration of flexible printed circuit sheet 232B, the base layer 244 can also include a thickness of approximately 0.01 mm, the conductive traces and pads 350, 358 can include a thickness of approximately 0.01-0.02 mm, and the insulative layer 364 can include a thickness of approximately 0.01 mm.

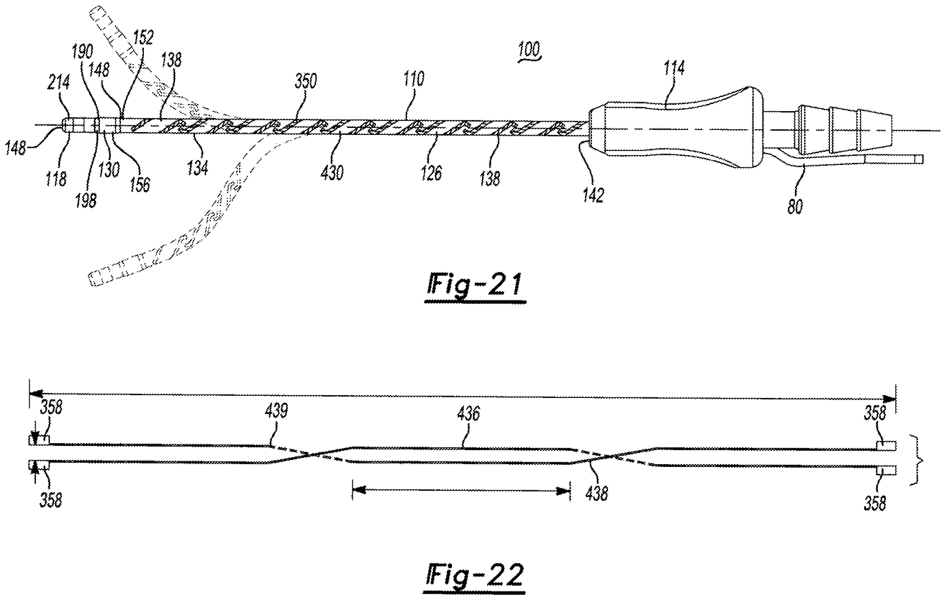

Referring now to Figure 21, an alternate surgical instrument 100 will be described in greater detail. Similar reference numerals will be used to describe similar structures shown in Figures 2-4. In one exemplary configuration, the surgical instrument 100 can be a malleable tool used for suction, including fluid and tissue removal in ENT procedures. Associated with the surgical instrument 100 is a flexible circuit 430 to transport electrical signals between the navigation probe interface 80 and a plurality of navigation coils 214. The flexible circuit 430 provides termination pads (not shown) for fine coil wires as well as cable wires and conductive traces 350 to bring electric connectivity to portions of the surgical instrument 100. As described further below, the conductive traces 350 are configured to minimize the pickup of stray electromagnetic noise.

The flexible circuit 430 described in detail below can be usable in other tracked medical devices or any other devices where tracking or navigating a distal tip of a device is desired. Thus, while the following description continues with reference to a navigated surgical instrument 100, the discussion is also applicable to the surgical instruments discussed above and any other appropriate instruments that require tracking or navigation of instruments that require substantially smooth exterior surfaces so as to not adversely interact with patient tissue. This is in contrast to existing instruments that have discrete wires wrapped around the outside of the instrument causing a ribbed effect. For example, the flexible circuit can be used in a micro coil based core tracker assembly, slanted coil based cranial stylets, biopsy needles, or other navigated instruments requiring challenging volumetric packaging constraints.

Surgical instrument 100 can include a tube assembly 110, a handle assembly 114, and a tracking sensor arrangement 118. Surgical instrument 100 or portions thereof can be configured for a single use such that it would be disposed of after such use. The tube assembly 110 can include a malleable elongated tubular body 126 and an insert portion 130. The malleable tubular body 126 can be formed from a malleable metallic material such that the tubular body 126 body portion can be bent between the proximal and distal ends from a first configuration to a second bent configuration and maintain the bent configuration.

The tubular body 126 can include an outer diameter 134 and an inner diameter 138 and can have a first or proximal end 142 coupled to the handle assembly 114 and a second opposite or distal end 148 configured to receive insert portion 130. As best seen in Figure 4, the second end 148 can include an internal annular recess 152 having an inner diameter 156 greater than the inner diameter 138 of the remaining portion of body 126. The body 126 can also be provided in various lengths and diameters including, by way of example, lengths from 50 mm - 500 mm and including 7, 9 and 12 French diameters (2.3, 3.0 and 4.0 mm). The insert portion 130 can be configured to provide non-malleable support for at least the tracking sensor 84.

Insert portion 130 can include a sleeve 190 received on an exterior thereof. Sleeve 190 can include an inner diameter 194 substantially equal to the outer diameter of insert portion 130, and an outer diameter 198 substantially equal to the outer diameter 134 of body 126. The insertion of the sleeve 190 into the first end 148 of the body can facilitate the electronic coupling of tracking coils with the navigation system. Alternatively, electronic coupling can be accomplished using soldering techniques. It should be appreciated that sleeve 190 can also be configured with different diameters relative to body 126.

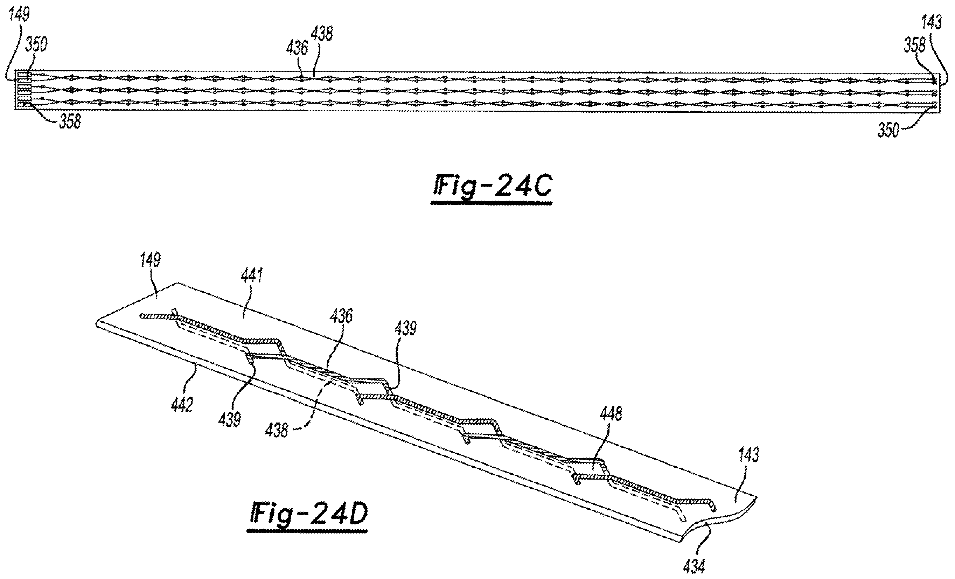

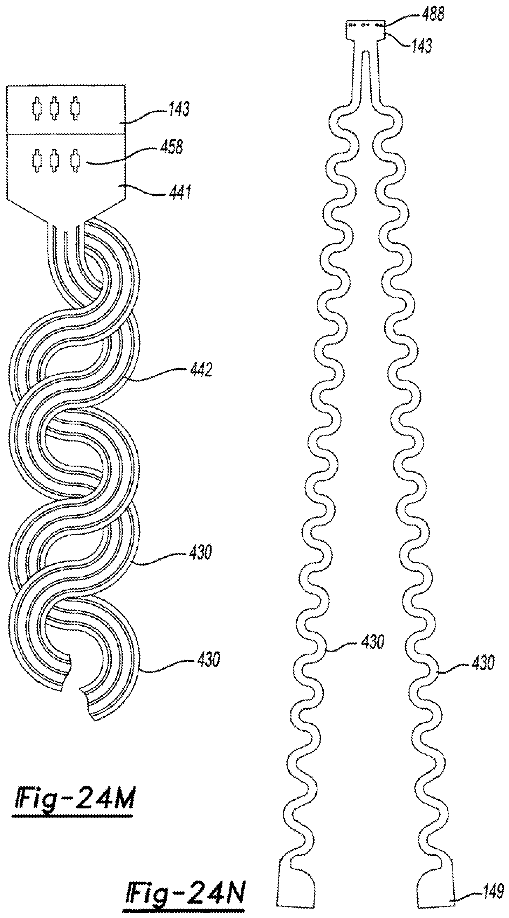

Figure 22 represents a top view of conductive traces 436, 438 associated with the flexible circuit 430 of Figure 21. The conductive traces 436, 438 are twisted to form loops configured to have opposite handedness from its nearest neighbors. The alternative handedness effectively cancels electromagnetic noise. In this regard, the twisted pairs minimize electromagnetic pickup that would degrade navigation performance. The twisted pair configuration is formed using through-substrate or base layer 434 connections, as is illustrated exemplarily in Figure 24D on the flexible base layer 434, and provides the needed form factor. In this regard, the flexible circuit 430 protrudes from the nominal surface radius of the body portion 126 by less than about .05 mm, thus minimizing interaction of the device with patient tissue. In other words, the thickness of the flex circuit is .08 mm which is less than the thickness of the twisted wires 0.4 to 0.5 mm. The conductive traces 436, 438 have first and second coupling pads 350 and 358 disposed at a first proximal end 142 and a second set of coupling pads 350 and 358 disposed at the second distal end 148, wherein the length of the flexible circuit 430 extending from the first proximal end 142 to the second distal end 148 is of the elongated body 126. Furthermore, the profile of the construction utilizing the flexible circuit 430 is substantially smoother and more uniform than the ribbed profile of the twisted wire configuration.

With particular reference to Figures 23A and 23B, the flexible circuit 430 having one or more, including three pairs of conductive traces 436, 438 as illustrated in Figure 22 is shown. The flexible circuit sheet comprises terminal pads 458. The flexible circuit 430 is generally disposed around the body portion 126 and extends from the tracking device 84 from the second distal end 148 to the first proximal end 142 and handle assembly 114. The flexible circuit is configured to conform to the bent configuration of the body portion 126 such that it does not strain or break during bending of the body portion 126. The flexible circuit 430 can be longitudinally or helically disposed around a portion of the body portion 126 from the tracking device 84 to the handle assembly 114, the helically disposed variant not forming part of the claimed invention. If longitudinally disposed, the flexible circuit is curved transverse to the longitudinal axis of the flexible circuit 430 to conform to the curvature of the instrument shaft, as shown in Figure 23A. The flexible circuit 430 can have a sinusoidal periphery or have a periphery formed of a plurality of curved line segments. If helically disposed, the flexible circuit 430 is wrapped helically around the longitudinal axis as is shown in Figure 21. To allow the body portion 126 to curve when the flexible circuit 430 is wound around the body portion 126, the flexible circuit can define gaps 433 which allow and accommodate for compression and tension of an external surface of the body portion 126. When the outer portion of the body portion 126 is in tension, the gaps 433 can expand, while the inner portion during the bending of the body portion 126.

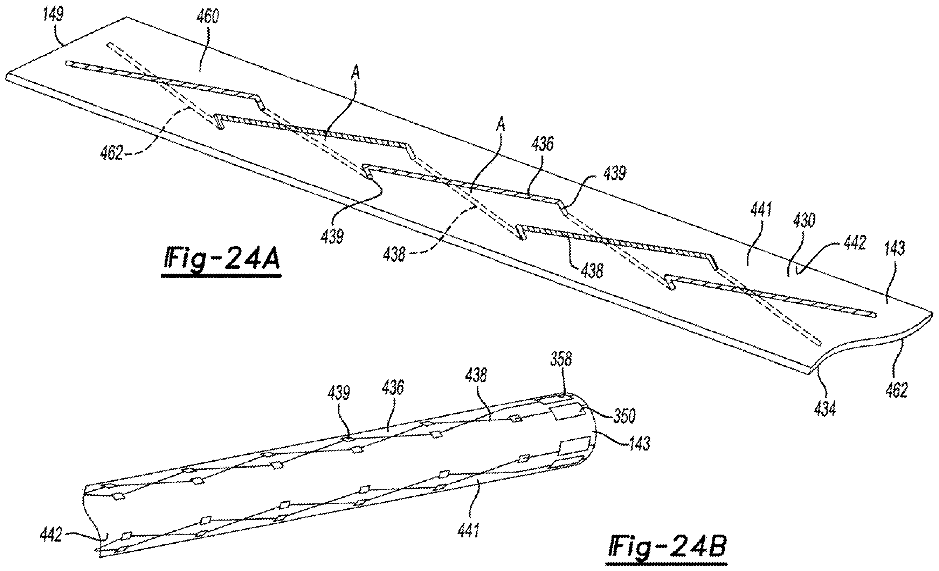

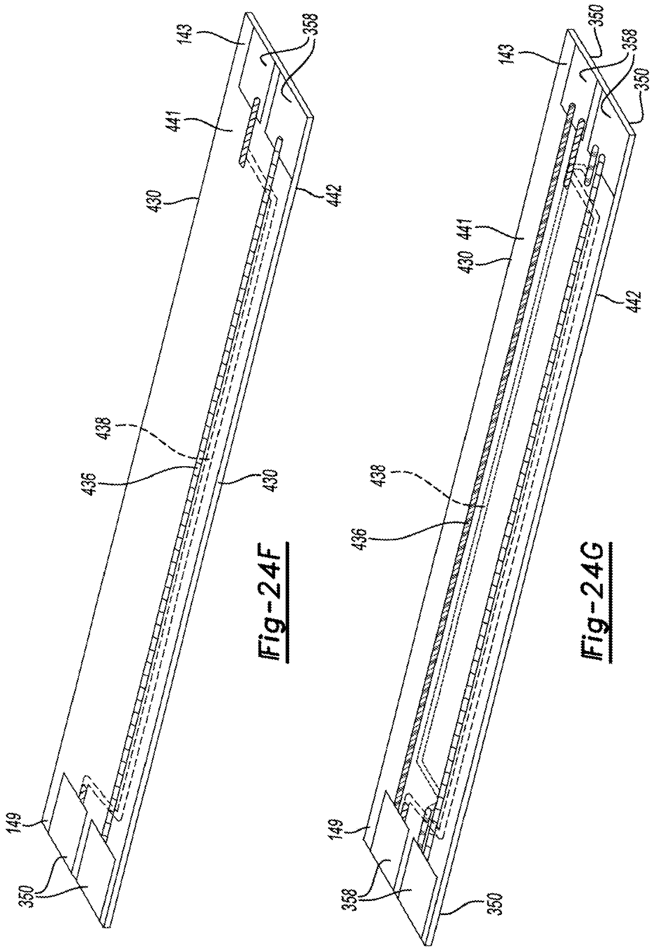

Figure 23A represents the malleable tube 110 shown in Figure 21. As shown, the flexible circuit 430 extends from the first proximal end 142 of the body portion 126 to the second distal end 148 of the body portion 126. A serpentine shaped flexible circuit 430 is laid longitudinally wrapped around the body portion 126. As described below, the conductors in the flexible circuit 430 can be generally parallel (see Figure 24F) or form twisted pairs (See Figure 24A) to allow the navigation system to properly account for electromagnetic noise. Each twist in the twisted pair produces a small loop to reduce electromagnetic coupling noise.

Figure 23B represents the flexible circuit 430 in a planar manner before it has been conformed as is shown in Figure 23A. The serpentine shaped flexed circuit 430, when wrapped around the body portion 126, defines the gaps 433 along edges of the flexible circuit. It is envisioned the maximum radius of curvature of the body portion 126 may be regulated by the size and positions of the defined gaps 433.

Figures 24A-24O represent perspective views of exemplary configurations of the flexible circuit 430 according to the present teachings. Generally, the flexible circuit 430 has the first and second conductive traces 436, 438 which are parallel or are twisted to reduce the influence of electromagnetic noise on the tracking system. The flexible circuit 430 can have a base layer 434 formed of a thin insulative material such as polyimide, polyethylene, terephthalate, latex, nitrile rubber, polysiloxanes, silicone, polyurethane, polyether block amide (trade name PEBAX), a first circuit trace 436 having a first upper portion 460 of the trace formed on a first upper side 441 of the base layer 434, and a second circuit trace 438 having a second lower portion 462 formed on an second lower side 442 of the base layer 434. The flexible circuit 430 can take any number of shapes which allow the flexible circuit 430 and medical device to bend.

The base layer 434 and insulative layers include material properties and a thickness configured to facilitate the flexible circuit 430 being flexible such that the flexible circuit 430 is adapted to conform to an exterior surface of the elongated body 126, as well as allow the elongated body 126 to bend along the longitudinal axis of the body portion 126. The flexible circuit 430 can have various components disposed between the proximal and distal ends 143, 149. In this regard, various electrical components such as amplifier or tracking coils can be attached. For example, coil assemblies 214 can be coupled to the flexible circuit 430 at predetermined locations between the proximal and distal end 143, 149. In other words, a single coil assembly can be located at the second distal end 149, multiple coils can be located along the length of the flexible circuit 430.

As shown in Figure 24A, the conductive traces 436, 438 can form a twisted pair configuration. The first trace 436 runs along the first upper side 441 and crosses over a second trace 438 positioned on an opposite second side 442 at an acute angle A. The first trace 436 then passes through the insulator or base layer 434 and runs along the second lower side 442. A second trace runs along the second lower side 442 and crosses over a first trace 436 positioned on the first upper side 441 at the acute angle A. The first and second conductive traces 436, 438 cross from over to under at electrical vias 439. The vias 439 extend transversely through the base layer 434 to connect the conductive trace from the first upper side 441 to the second lower side 442.

Figures 24B and 24C represent a flexible circuit 430 having three twisted pair configurations as shown in Figure 24A as they would be wrapped around an elongated body 126. As shown, coupling pads 358 are provided for coupling to the leads of three coils at the second distal end 149 or to the coupling cable wires at the first proximal end 143. The vias 439 are disposed between the overlapping conductive traces 436, 438 were the traces are located on opposite sides of the base layer 434. Generally, the conductive traces 436, 438 are positioned adjacent and parallel to each other to minimize conductor loop size.

As shown in Figure 24D, the loop area 446 can be reduced by placing the first trace 436 directly over the second trace 438 on the base layer 434 for a majority of the length of the trace except where the conductive traces separate to form vias 439 in passing areas 448. In other words, for short distances, the conductive traces separate enough to allow the traces to pass through the base layer 434 to the other side. At this point, the traces return to a position where they are parallel to each other. The loop area can further be reduced by reducing the thickness of the insulative base layer 434. It is envisioned the base layer 434 can have a thickness of about 0.025 mm. The base layer 434 can be formed of polyimide, polyethylene, terephthalate, as well as a thin elastic insulative base layer 434. The base layers 434 can be latex, nitrile rubber, polysiloxanes, silicone, polyurethane, polyether block amide or PEBAX.

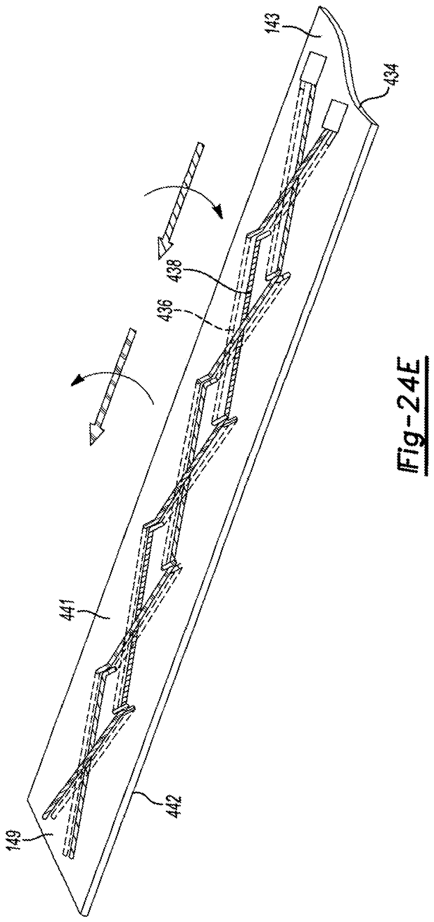

As shown in Figure 24E, by using multiple base or insulative layers, and placing oppositely handed twisted pairs intertwisted with an adjacent pair of conductors, further noise cancellation can be accomplished. The oppositely handed twisted pairs connect in parallel, adding redundancy to this canceled double twisted pair configuration. As previously discussed, conductive traces 436, 438 are periodically passed through from a first upper side 441 of the base layer 434 to a second lower side 442 of the base layer 434 to form the twisted configuration. As can be see, up to four conductive traces 436, 438 can be separated by insulative layers and run in parallel with the second pair being a twisted pair with opposite handedness to the first pair of conductive traces. These conductive traces are shuttled through various based layers 434 to form the twisted pair constructions.

As shown in Figures 24F and 24G, the pair conductive traces 436, 438 need not be in a twisted pair configuration. Figure 24F depicts a noise minimizing parallel pair set. The configuration uses the thinness of the base layer to minimize noise. Figure 24G depicts a double oppositely oriented parallel pair with the second pair being a twisted pair with opposite handedness to the first pair of conductive traces. The proximal end 143 of the flexible circuit 430 has a pair of coupling pads 358. The coupling pads 358 are coupled to a pair of parallel conductive traces 436, 438 which are directly over one another, each disposed on opposing sides 441, 442 of the base layer 434. At the distal end 149 of the base layer 434, another pair of coupling pads 358 are provided to couple the flexible circuit to the tracking coils or tracking device 84. In situations where there are numerous conductive traces on the same base layer 434, coupling pads can be found on first and second (top or bottom) sides of the base layer 434. This allows for the convenient coupling of tracking devices to the base layer 434 using soldering or connectors.

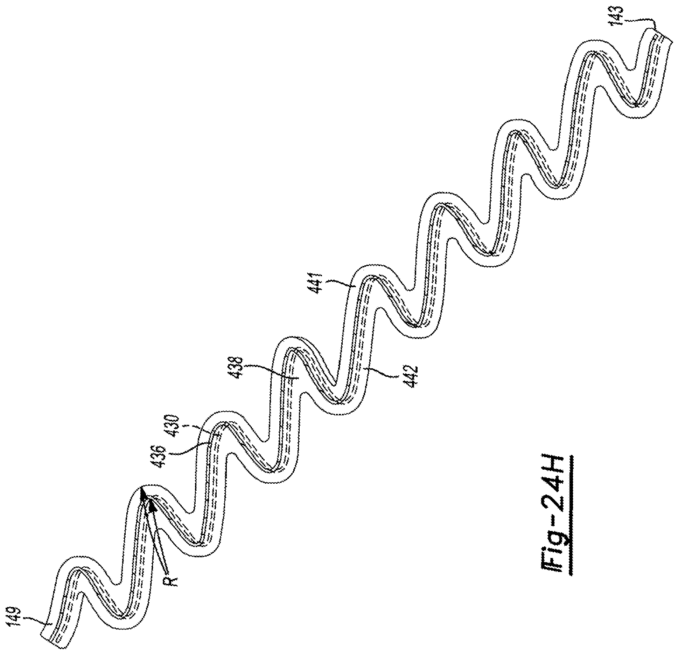

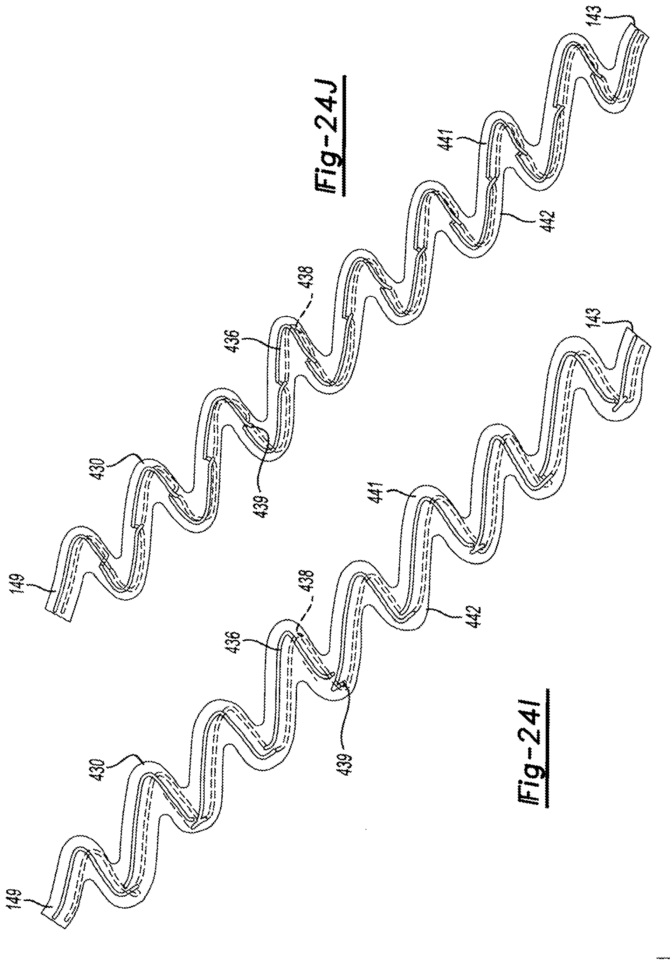

As shown in Figures 24H-24N, the flexible circuits can have a sinusoidal form. Each conductive trace 436, 438 can be defined on a single side of the base layer 434 and can have radius of curvature R which is between the outer and inner radiuses of curvature of the base layer 434. Alternatively, as shown in Figures 24I and 24J, the pairs of conductive traces can be formed in twisted pairs by alternating position on opposite sides of the flexible circuit as described above. The pair crossovers or vias 349 occur at locations along the curve. Generally, the vias 349 are positioned transverse to the longitudinal axis of the body portion 126 or base layer 434. As shown in Figure 24J, the vias occur at sinusoidal midpoints wherein at Figure 24I the vias occur at sinusoidal minimums or maximums. The vias 349 are between peaks and valleys in the sinusoidal substrate in order to minimize the mechanical stresses on the electrical vias 349. Generally, the through paths or vias 349 will be formed perpendicular to the longitudinal axis of the tool.

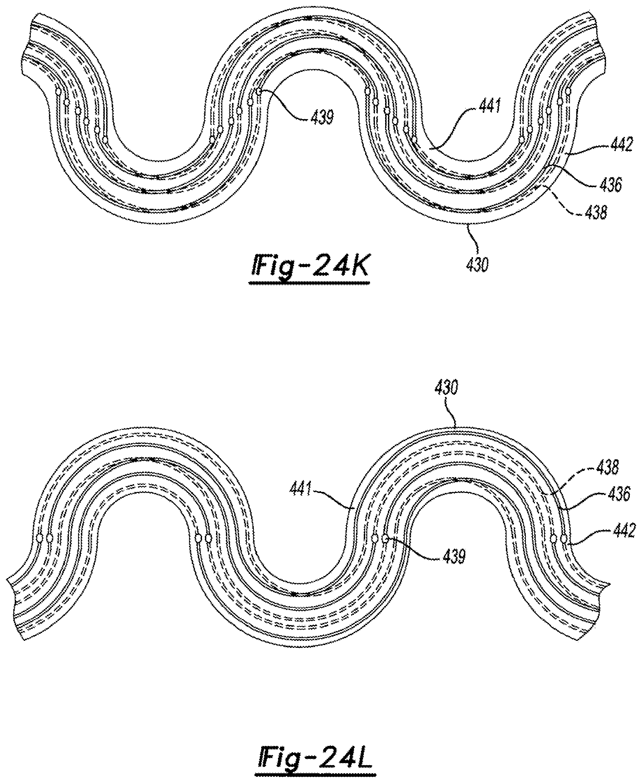

As shown in Figures 24K and 24L, three pair of conductive traces can be formed on a single serpentine or sinusoidal shaped base layer 434. In other words, the conductor traces 436, 438 can be three pairs of conductive traces in a single serpentine flexible circuit 430. As can be seen, the position of the vias 439 can be staggered along the length of the flexible circuit so each new curvature represents a new location for the via 439. Alternatively, the vias 439 can be staggered adjacent to the centerline of the flexible circuit.

As shown in Figures 24M-24N, a pair of serpentine flexible circuits 430 can be braided together to form a flat twisted pair by combining two individual sinusoidal patterned base layers 434, each with three sinusoidally patterned traces explicitly braided into three flat pairs. The base layers 434 can be joined at the proximal end 142 to facilitate the coupling of the tracking device 84. The use of several flexible circuits 430 in this configuration can provide a proper level of rigidity of the body portion 126.

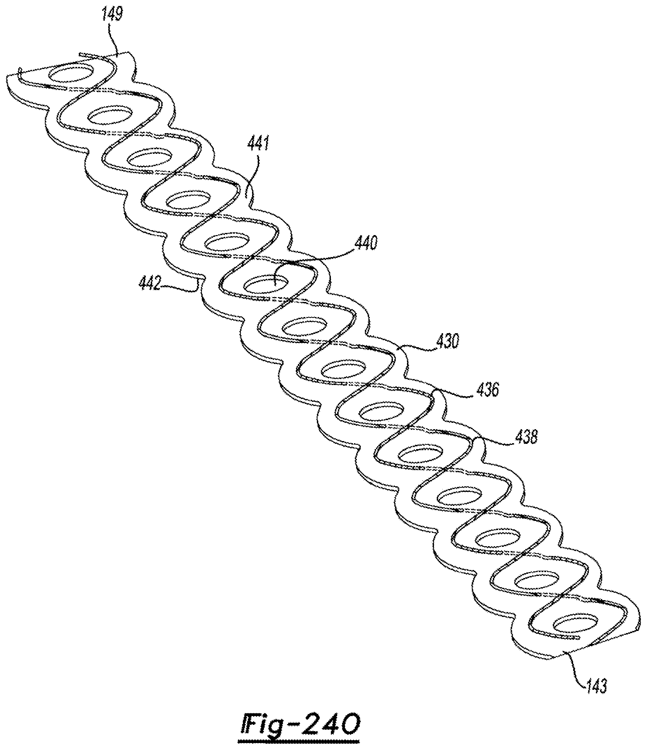

Figure 24O represents an alternate flexible circuit 430 having a braided pair configuration. The leads 436, 438 are disposed through the base layer and wrapped around apertures 440 defined in the base layer 344. As shown, the base layers 434 form the braided pair configuration and can alternate sides of the flexible circuit 430 as described above.

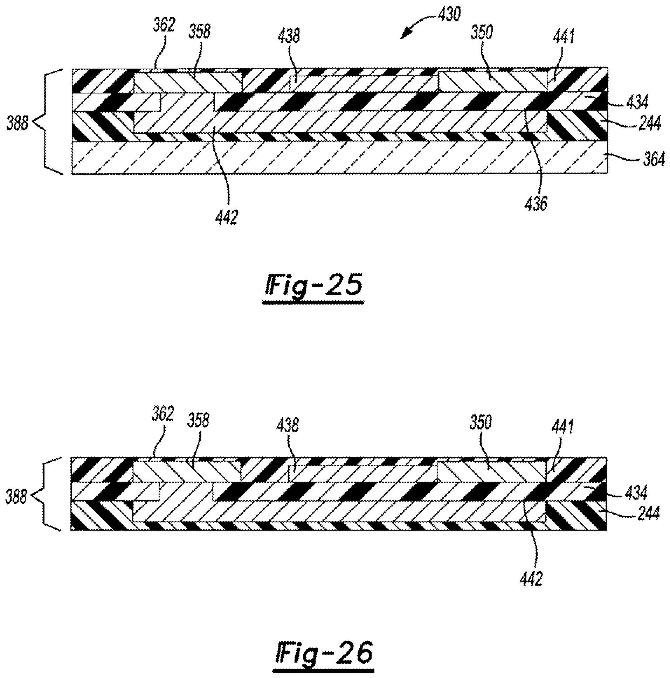

Referring to Figure 25, a cross-sectional view of the flexible circuit 430 is shown in a configuration utilizing a pressure sensitive adhesive 364. In this configuration, the base layer 434 can include a thickness of approximately 0.01 mm, the conductive traces 436, 438 and pads 350, 358 can include a thickness of approximately 0.04 mm, and the insulative layer 362 can include a thickness of approximately 0.02 mm. In the assembled configuration, the flexible printed circuit sheet can include an overall thickness 388 of approximately 0.07 mm without adhesive 364 and an overall thickness 388 of 0.11 mm with adhesive 364.

With reference to Figure 26, the flexible circuit 430 is shown having a smaller overall thickness 388 of approximately 0.05 mm without adhesive 364, and an overall thickness 388 of 0.07 mm with adhesive 364. In this configuration, the base layer 244 can similarly have a thickness of approximately 0.01 mm, the conductive traces 436, 438 and pads 350, 358 can include a thickness of approximately 0.02 - 0.03 mm, and the insulative layer can include a thickness of approximately 0.01 mm.

It will be appreciated that while various configurations of flexible printed circuit sheets have been discussed herein, other configurations can be utilized taking advantage of the thin, compact and conformable features of such flexible printed circuit sheets. In this exemplary configuration, such a flexible printed circuit sheet could include a length configured to be helically wound from the distal end 148 to the handle assembly 114.

While one or more specific examples have been described and illustrated, it will be understood by those skilled in the art that various changes may be made and equivalence may be substituted for elements thereof without departing from the scope of the present teachings as defined in the claims. Furthermore, the mixing and matching of features, elements and/or functions between various examples may be expressly contemplated herein so that one skilled in the art would appreciate from the present teachings that features, elements and/or functions of one example may be incorporated into another example as appropriate, unless described otherwise above. Moreover, many modifications may be made to adapt a particular situation or material to the present invention without departing from the scope thereof, which is defined by the claims.

## Claims

1. A surgical instrument, comprising:
an elongated body (126) extending from a proximal end to a distal end;
a tracking device (84) disposed at the distal end; and
a flexible circuit sheet (430 - Figure 23A) attached to the elongated body and extending from the proximal end to the distal end of the elongated body
wherein,
the flexible circuit sheet is a first flexible circuit sheet that has a sinusoidal periphery that is longitudinally disposed along a same side of a portion of the elongated body and that is curved to conform to a curvature of the elongated body;
the flexible circuit sheet has a base layer (244), first and second conductive traces (350) on the base layer, and an insulative layer (362) formed over the first and second traces,
the first trace and the second trace are coupled to the tracking device,
the first trace and the second trace extend from the proximal end to the distal end, the sinusoidal periphery defining gaps (433) to allow and accommodate for compression and tension of an external surface of the elongated body during bending of the elongated body.

2. The surgical instrument of claim 1, wherein the flexible circuit sheet protrudes from a surface of the elongated body by less than 0.08 mm.

3. The surgical instrument of claim 1 or 2, wherein the first and second conductive traces comprises a first pair of terminal pads (458) at the proximal end.

4. The surgical instrument of any one of claims 1 to 3, wherein the first and second conductive traces form one of a twisted pair, a double oppositely oriented parallel pair or double oppositely oriented twisted pair.

5. The surgical instrument of any one of claims 1 to 4, wherein the flexible circuit sheet has features configured to allow the flexible circuit to bend, the features selected from the group consisting of a sinusoid peripheral shape, a sinusoidal base curve peripheral shape, a base layer formed of an elastic PABAX, and combinations thereof.

6. The surgical instrument of any one of claims 1 to 5, wherein the first and second conductive traces have a first portion of the first and second conductive traces formed on a first side of the base layer and a second portion of the first and second conductive traces formed on an opposed second side of the base layer;
the insulative layer formed over the first and second conductive traces to isolate the first and second conductive traces from an external environment, wherein the base layer, first and second conductive traces and insulative layer form the flexible circuit sheet; and
wherein the base layer and the insulative layer include material properties and a thickness configured to facilitate the flexible circuit sheet being flexible such that the flexible circuit sheet is adapted to conform to an exterior surface of the elongated body.

7. The surgical instrument of any one of claims 1 to 6, further comprising an outer polymeric shrink fit layer covering the elongated body and flexible circuit sheet.

8. The surgical instrument of any one of claims 1 to 7, further comprising an adhesive (364) configured to contact a lower side of the flexible circuit sheet and adapted to adhere the flexible circuit sheet to a portion of the elongated body.

9. The surgical instrument of claim 8, wherein the flexible circuit sheet and the adhesive have a combined thickness of between 0.10 and 0.12 mm.

10. The surgical instrument of any one of claims 1 to 7, wherein a portion of the elongated body is malleable such that it can be bent into a plurality of shapes; and
wherein the flexible circuit sheet is adhered to a cylindrical outer surface of the elongated body and is configured to change shape in response to bending of a portion of the elongated body .

11. The surgical instrument of any one of claims 1 to 6, further comprising an outer polymeric shrink fit layer covering the elongated body and flexible circuit sheet, the flexible circuit being moveably captured between the outer polymeric shrink layer and the elongated body .

12. The surgical instrument of any one of claims 1 to 11, wherein the tracking device includes a plurality of tracking coils configured to nest around a generally cylindrical surface of the body , wherein the plurality of tracking coils includes three coil assemblies each having a respective pair of lead wires coupled to corresponding conductive traces of the flexible circuit sheet.

13. The surgical instrument of claim 1, comprising:
the elongated body having a tubular body extending from the proximal end to the distal end, the elongated tubular body having an inner diameter defining a first internal flow passage between the proximal end and the distal end, and formed of a malleable deformable material such that the elongated tubular body can be bent between the proximal end and the distal end from a first configuration to a second bent configuration;
the tracking device coupled to the elongated tubular body adjacent to the distal end, the tracking device adapted to cooperate with a navigation system to track the distal end of the instrument, the tracking device including the flexible circuit sheet having the base layer, the first and second conductive traces each having a first portion of the plurality of conductive traces formed on a first side of the base layer and a second portion formed on an opposite second side of the base layer and a pair of lead wires for each coil assembly coupled to corresponding conductive traces; and
the insulative layer formed over the conductive traces to isolate the conductive traces from an external environment, the base layer, the first and second conductive traces and insulative layer form the flexible circuit sheet, wherein the base layer and an insulative layer include material properties and a thickness configured to facilitate the flexible circuit sheet being flexible such that the flexible circuit sheet is adapted to conform to an exterior surface of the elongated tubular body.

14. The surgical instrument of any one of claims 1 to 13, further comprising a second flexible circuit sheet extending from the proximal end to the distal end of the elongated body,
wherein the second flexible circuit sheet comprises a third trace and a fourth trace, has a sinusoidal periphery, and is braided with the first flexible circuit sheet.

15. The surgical instrument of any one of claims 1 to 14, further comprising:
a first portion of the first trace is on a first side of the base layer,
a second portion of the first trace is on a second side of the base layer,
a first portion of the second trace is on the first side of the base layer, and
a second portion of the second trace is on the second side of the base layer.

## Patentansprüche

1. Chirurgisches Instrument, umfassend:
einen verlängerten Körper (126), der sich von einem proximalen Ende bis zu einem distalen Ende erstreckt;
eine Verfolgungsvorrichtung (84), die an dem distalen Ende angeordnet ist; und
eine flexible Schaltplatte (430 - Figur 23A), die an dem verlängerten Körper befestigt ist und sich von dem proximalen Ende bis zu dem distalen Ende des verlängerten Körpers erstreckt, wobei
die flexible Schaltplatte eine erste flexible Schaltplatte ist, die einen sinusförmigen Umfang aufweist, die längs entlang derselben Seite eines Abschnitts des verlängerten Körpers angeordnet und gebogen ist, um sich an eine Wölbung des verlängerten Körpers anzupassen;
wobei die flexible Schaltplatte eine Basisschicht (244), erste und zweite leitfähige Abtastspuren (350) an der Basisschicht, und eine isolierende Schicht (362), die über den ersten und zweiten Abtastspuren gebildet ist, aufweist,
wobei die erste Abtastspur und die zweite Abtastspur an die Verfolgungsvorrichtung gekoppelt sind,
wobei sich die erste Abtastspur und die zweite Abtastspur von dem proximalen Ende bis zu dem distalen Ende erstrecken, wobei der sinusförmige Umfang Aussparungen (433) definiert, um ein Verdichten und eine Spannung einer Außenfläche des verlängerten Körpers während dem Biegen des verlängerten Körpers zu erlauben und aufzunehmen.

2. Chirurgisches Instrument nach Anspruch 1, wobei die flexible Schaltplatte von einer Fläche des verlängerten Körpers um weniger als 0,08 mm hervorsteht.

3. Chirurgisches Instrument nach Anspruch 1 oder 2, wobei die erste und die zweite leitfähige Abtastspur jeweils ein erstes Paar von Anschlussflächen (458) an dem proximalen Ende umfasst.

4. Chirurgisches Instrument nach einem der Ansprüche 1 bis 3, wobei die ersten und zweiten leitfähigen Abtastspuren eines von einem gewundenen Paar, einem entgegengesetzt orientierten, parallelen Doppelpaar oder einem entgegengesetzt orientierten, gewundenen Doppelpaar bilden.

5. Chirurgisches Instrument nach einem der Ansprüche 1 bis 4, wobei die flexible Schaltplatte Merkmale aufweist, die konfiguriert sind, um es der flexiblen Schaltung zu erlauben, sich zu biegen, wobei die Merkmale ausgewählt sind aus der Gruppe bestehend aus einer sinusförmigen Umfangsform, einer sinusförmigen Basiskurven-Umfangsform, einer Basisschicht, die aus einem elastischen PABAX gebildet ist, sowie Kombinationen davon.

6. Chirurgisches Instrument nach einem der Ansprüche 1 bis 5, wobei die ersten und zweiten leitfähigen Abtastspuren einen ersten Abschnitt der ersten und zweiten leitfähigen Abtastspuren aufweisen, der an einer ersten Seite der Basisschicht gebildet ist, und einen zweiten Abschnitt der ersten und zweiten leitfähigen Abtastspuren, der an einer gegenüberliegenden zweiten Seite der Basisschicht gebildet ist;
wobei die isolierende Schicht über den ersten und zweiten leitfähigen Abtastspuren gebildet ist, um die ersten und zweiten leitfähigen Abtastspuren vor einer externen Umgebung zu isolieren, wobei die Basisschicht, die ersten und zweiten leitfähigen Abtastspuren und die isolierende Schicht eine flexible Schaltplatte bilden; und
wobei die Basisschicht und die isolierende Schicht Materialmerkmale und eine Dicke beinhalten, die konfiguriert sind, um die flexible Schaltplatte aufzunehmen, die derart flexibel ist, dass die flexible Schaltplatte adaptiert ist, um sich an die Außenfläche des verlängerten Körpers anzupassen.

7. Chirurgisches Instrument nach einem der Ansprüche 1 bis 6, ferner umfassend eine Schrumpfsitz-Polymerschicht, die den verlängerten Körper und die flexible Schaltplatte bedeckt.

8. Chirurgisches Instrument nach einem der Ansprüche 1 bis 7, ferner umfassend einen Klebstoff (364), der konfiguriert ist, um eine Unterseite der flexiblen Schaltplatte zu kontaktieren und angepasst ist, um die flexible Schaltplatte an einen Abschnitt des verlängerten Körpers anzuhaften.

9. Chirurgisches Instrument nach Anspruch 8, wobei die flexible Schaltplatte und der Klebstoff eine kombinierte Dicke von zwischen 0,10 und 0,12 mm aufweisen.

10. Chirurgisches Instrument nach einem der Ansprüche 1 bis 7, wobei ein Abschnitt des verlängerten Körpers derart verformbar ist, dass er in eine Vielzahl von Formen gebogen werden kann; und
wobei die flexible Schaltplatte an eine zylindrische Außenfläche des verlängerten Körpers angehaftet wird und konfiguriert ist, um die Form als Reaktion auf das Verbiegen eines Abschnitts des verlängerten Körpers zu verändern.

11. Chirurgisches Instrument nach einem der Ansprüche 1 bis 6, ferner umfassend eine äußere Schrumpfsitz-Polymerschicht, die den verlängerten Körper und die flexible Schaltplatte bedeckt, wobei die flexible Schaltplatte zwischen der äußeren Schrumpfsitz-Polymerschicht und dem verlängerten Körper beweglich erfasst ist.

12. Chirurgisches Instrument nach einem der Ansprüche 1 bis 11, wobei die Verfolgungsvorrichtung eine Vielzahl von Verfolgungsspulen beinhaltet, die konfiguriert sind, um sich um eine im Allgemeinen zylindrische Fläche des Körpers herum zu verschachteln, wobei die Vielzahl von Verfolgungsspulen drei Spulenbaugruppen beinhaltet, die jeweils ein entsprechendes Paar von Verbindungsleitungen aufweisen, die an korrespondierende leitfähige Abtastspuren der flexiblen Schaltplatte gekoppelt sind.

13. Chirurgisches Instrument nach Anspruch 1, umfassend:
einen verlängerten Körper, der einen rohrförmigen Körper aufweist, der sich von dem proximalen Ende bis zu dem distalen Ende erstreckt, wobei der verlängerte, rohrförmige Körper einen Innendurchmesser aufweist, der einen ersten internen Strömungsdurchgang zwischen dem proximalen Ende und dem distalen Ende definiert und aus einem verformbaren, deformierbaren Material gebildet ist, sodass der verlängerte, rohrförmige Körper zwischen dem proximalen Ende und dem distalen Ende von einer ersten Konfiguration in eine zweite, gebogene Konfiguration gebogen werden kann;
eine Verfolgungsvorrichtung, die an den verlängerten, rohrförmigen Körper benachbart des distalen Endes gekoppelt ist, wobei die Verfolgungsvorrichtung angepasst ist, um mit einem Navigationssystem zu kooperieren, um das distale Ende des Instruments zu verfolgen, wobei die Verfolgungsvorrichtung die flexible Schaltplatte, welche die Basisschicht aufweist, beinhaltet, wobei die ersten und zweiten leitfähigen Abtastspuren jeweils einen ersten Abschnitt der Vielzahl von leitfähigen Abtastspuren aufweisen, die an einer ersten Seite der Basisschicht gebildet ist, und einen zweiten Abschnitt, der an einer gegenüberliegenden zweiten Seite der Basisschicht gebildet ist, und ein Paar von Verbindungsleitungen für jede Spulenbaugruppe, die an die korrespondierenden leitfähigen Abtastspuren gekoppelt sind; und
eine leitfähige Schicht, die über den leitfähigen Abtastspuren gebildet ist, um die leitfähigen Abtastspuren von einer externen Umgebung zu isolieren, wobei die Basisschicht, die ersten und zweiten leitfähigen Abtastspuren und die isolierende Schicht die flexible Schaltplatte bilden, wobei die Basisschicht und eine isolierende Schicht Materialmerkmale und eine Dicke beinhalten, die konfiguriert sind, um die flexible Schaltplatte aufzunehmen, die derart flexibel ist, dass die flexible Schaltplatte adaptiert ist, um sich an eine Außenfläche des verlängerten Körpers anzupassen.

14. Chirurgisches Instrument nach einem der Ansprüche 1 bis 13, ferner umfassend eine zweite flexible Schaltplatte, die sich von dem proximalen Ende bis zu dem distalen Ende des verlängerten Körpers erstreckt,
wobei die zweite flexible Schaltplatte eine dritte Abtastspur und eine vierte Abtastspur umfasst, einen sinusförmigen Umfang aufweist und mit der ersten flexiblen Schaltplatte verflochten ist.

15. Chirurgisches Instrument nach einem der Ansprüche 1 bis 14, ferner umfassend:
einen ersten Abschnitt der ersten Abtastspur, der sich an einer ersten Seite der Basisschicht befindet,
einen zweiten Abschnitt der ersten Abtastspur, der sich an einer zweiten Seite der Basisschicht befindet,
einen ersten Abschnitt der zweiten Abtastspur, der sich an der ersten Seite der Basisschicht befindet, und
einen zweiten Abschnitt der zweiten Abtastspur, der sich an der zweiten Seite der Basisschicht befindet.

## Revendications

1. Instrument chirurgical comprenant :
un corps allongé (126) s'étendant à partir d'une extrémité proximale vers une extrémité distale ;
un dispositif de suivi (84) disposé au niveau de l'extrémité distale ; et
une feuille de circuit souple (430 - figure 23A) fixée au corps allongé et s'étendant à partir de l'extrémité proximale vers l'extrémité distale du corps allongé,
dans lequel
la feuille de circuit souple est une première feuille de circuit souple qui a une périphérie sinusoïdale qui est disposée longitudinalement le long d'un même côté d'une partie du corps allongé et qui est courbée pour être conforme à une courbure du corps allongé ;
² la feuille de circuit souple a une couche de base (244), des première et deuxième traces conductrices (350) sur la couche de base, et une couche isolante (362) formée sur les première et deuxième traces,
la première trace et la deuxième trace sont couplées au dispositif de suivi,
la première trace et la deuxième trace s'étendent à partir de l'extrémité proximale vers l'extrémité distale, la périphérie sinusoïdale définissant des espaces (433) pour permettre et s'adapter à la compression et la tension d'une surface externe du corps allongé au cours du pliage du corps allongé.

2. Instrument chirurgical selon la revendication 1, dans lequel la feuille de circuit souple fait saillie à partir d'une surface du corps allongé à raison de moins de 0,08 mm.

3. Instrument chirurgical selon la revendication 1 ou 2, dans lequel les première et deuxième traces conductrices comprennent une première paire de plots de borne (458) au niveau de l'extrémité proximale.

4. Instrument chirurgical selon l'une quelconque des revendications 1 à 3, dans lequel les première et deuxième traces conductrices forment une paire torsadée, une double paire parallèle à orientation opposée ou une double paire torsadée à orientation opposée.

5. Instrument chirurgical selon l'une quelconque des revendications 1 à 4, dans lequel la feuille de circuit souple a des caractéristiques configurées pour permettre au circuit souple de se plier, les caractéristiques étant sélectionnées dans le groupe constitué d'une forme périphérique sinusoïdale, d'une forme périphérique incurvée à base sinusoïdale, d'une couche de base formée d'un PABAX élastique, et des combinaisons de celles-ci.

6. Instrument chirurgical selon l'une quelconque des revendications 1 à 5, dans lequel les première et deuxième traces conductrices ont une première partie des première et deuxième traces conductrices formée sur un premier côté de la couche de base et une seconde partie des première et deuxième traces conductrices formée sur un second côté opposé de la couche de base ;
la couche isolante étant formée sur les première et deuxième traces conductrices pour isoler les première et deuxième traces conductrices à partir d'un environnement externe, dans lequel la couche de base, les première et deuxième traces conductrices et la couche isolante forment la feuille de circuit souple ; et
dans lequel la couche de base et la couche isolante comprennent des propriétés matérielles et une épaisseur conçues pour permettre à la feuille de circuit souple d'être souple de sorte que la feuille de circuit souple est conçue pour être conforme à une surface extérieure du corps allongé.

7. Instrument chirurgical selon l'une quelconque des revendications 1 à 6, comprenant en outre une couche d'ajustement rétractable polymère externe recouvrant le corps allongé et la feuille de circuit souple.

8. Instrument chirurgical selon l'une quelconque des revendications 1 à 7, comprenant en outre un adhésif (364) configuré pour venir en contact avec un côté inférieur de la feuille de circuit souple et conçu pour coller la feuille de circuit souple à une partie du corps allongé.

9. Instrument chirurgical selon la revendication 8, dans lequel la feuille de circuit souple et l'adhésif ont une épaisseur combinée comprise entre 0,10 et 0,12 mm.

10. Instrument chirurgical selon l'une quelconque des revendications 1 à 7, dans lequel une partie du corps allongé est malléable de sorte qu'elle peut être pliée en une pluralité de formes ; et
dans lequel la feuille de circuit souple est collée à une surface externe cylindrique du corps allongé et est configurée pour modifier la forme en réponse au pliage d'une partie du corps allongé.

11. Instrument chirurgical selon l'une quelconque des revendications 1 à 6, comprenant en outre une couche d'ajustement rétractable polymère externe recouvrant le corps allongé et la feuille de circuit souple, le circuit souple étant capturé de manière mobile entre la couche rétractable polymère externe et le corps allongé.

12. Instrument chirurgical selon l'une quelconque des revendications 1 à 11, dans lequel le dispositif de suivi comprend une pluralité de bobines de suivi configurées pour se loger autour d'une surface généralement cylindrique du corps, dans lequel la pluralité de bobines de suivi comprend trois ensembles bobine ayant chacun une paire de fils conducteurs respective couplée aux traces conductrices correspondantes de la feuille de circuit souple.

13. Instrument chirurgical selon la revendication 1 comprenant :
le corps allongé ayant un corps tubulaire s'étendant à partir de l'extrémité proximale vers l'extrémité distale, le corps tubulaire allongé ayant un diamètre interne définissant un premier passage d'écoulement interne entre l'extrémité proximale et l'extrémité distale, et constitué d'un matériau malléable déformable de sorte que le corps tubulaire allongé peut être plié entre l'extrémité proximale et l'extrémité distale à partir d'une première configuration vers une seconde configuration pliée ;
le dispositif de suivi couplé au corps tubulaire allongé adjacent à l'extrémité distale, le dispositif de suivi conçu pour coopérer avec un système de navigation pour suivre l'extrémité distale de l'instrument, le dispositif de suivi comprenant la feuille de circuit souple ayant la couche de base, les première et deuxième traces conductrices ayant chacune une première partie de la pluralité de traces conductrices formée sur un premier côté de la couche de base et une seconde partie formée sur un second côté opposé de la couche de base et une paire de fils conducteurs pour chaque ensemble bobine couplée aux traces conductrices correspondantes ; et
la couche isolante formée sur les traces conductrices pour isoler les traces conductrices d'un environnement externe, la couche de base, les première et deuxième traces conductrices et la couche isolante forment la feuille de circuit souple, la couche de base et une couche isolante comprenant des propriétés matérielles et une épaisseur configurée pour permettre à la feuille de circuit souple d'être souple de sorte que la feuille de circuit souple est conçue pour être conforme à une surface externe du corps tubulaire allongé.

14. Instrument chirurgical selon l'une quelconque des revendications 1 à 13, comprenant en outre une seconde feuille de circuit souple s'étendant à partir de l'extrémité proximale vers l'extrémité distale du corps allongé,
dans lequel la seconde feuille de circuit souple comprend une troisième trace et une quatrième trace, a une périphérie sinusoïdale et est tressée avec la première feuille de circuit souple.

15. Instrument chirurgical selon l'une quelconque des revendications 1 à 14, comprenant en outre :
une première partie de la première trace qui est sur un premier côté de la couche de base,
une seconde partie de la première trace qui est sur un second côté de la couche de base,
une première partie de la deuxième trace qui est sur le premier côté de la couche de base, et
une seconde partie de la deuxième trace qui est sur le second côté de la couche de base.
